# EUROPÄISCHE PATENTSCHRIFT

(11) **EP 0 681 713 B1**
(45) Veröffentlichungstag und Bekanntmachung des Hinweises auf die Patenterteilung: **18.11.1998**
(21) Anmeldenummer: 93921907.7
(22) Anmeldetag: 04.10.1993
(51) Int. Cl.: G03F 7/029, G03F 7/004, G03F 7/039, G03F 7/075, C07C 309/63

(54) **SULFONSÄUREESTER, DAMIT HERGESTELLTE STRAHLUNGSEMPFINDLICHE GEMISCHE UND DEREN VERWENDUNG**
SULPHONIC ACID ESTERS, RADIATION-SENSITIVE MIXTURES MADE THEREWITH, AND THEIR USE
ESTERS D'ACIDE SULFONIQUE, MELANGES RADIOSENSIBLES REALISES AVEC, ET LEUR UTILISATION

(30) Priorität: 01.02.1993 DE 4302681
(43) Veröffentlichungstag der Anmeldung: 15.11.1995
(73) Patentinhaber: Clariant GmbH, 65929 Frankfurt am Main (DE)
(72) Erfinder: PAWLOWSKI, Georg, D-65187 Wiesbaden (DE); SPIESS, Walter, D-64807 Dieburg (DE); RÖSCHERT, Horst, D-55437 Ober-Hilbersheim (DE); APPEL, Wolfgang, D-65779 Kelkheim (DE); HERR, Walter, D-65817 Eppstein (DE)
(86) Internationale Anmeldenummer: EP9302701
(87) Internationale Veröffentlichungsnummer: WO9418606

(56) Entgegenhaltungen:
- EP-A- 0 497 342
- EP-A- 0 510 444
- EP-A- 0 510 447
- JOURNAL OF ORGANIC CHEMISTRY Nr. 14, 6. Juli 1990, Seiten 4448 - 4454 D.W. REYNOLDS ET AL. 'Exploring the Chemistry of the 2-Arylhexafluoro-2-propanol Group: Synthesis and Reactions of a New Highly Fluorinated Monomer Intermediate and Its Derivatives' in der Anmeldung erw hnt
- JOURNAL OF THE AMERICAN CHEMICAL SOCIETY Nr. 13, 22. Juni 1977, Seiten 4400 - 4404 G.W. ASTROLOGES, G.W. MARTIN 'Reactions of Trialkoxysulfuranes (Orthosulfinates) with Trifluoromethanesulfonic Acid' in der Anmeldung erw hnt
- JOURNAL OF THE AMERICAN CHEMICAL SOCIETY Nr. 12, 11. Juni 1986, Seiten 3470 - 3474 A.D. ALLEN ET AL. 'Doubly Destabilized Carbocations. Strong Aryl Delocalization and the Attenuation of Rate Decelerating Effects of CF3 and CN Groups' in der Anmeldung erw hnt

## Beschreibung

Die Erfindung betrifft Sulfonsäureester und damit hergestellte strahlungsempfindliche, positiv oder negativ arbeitende Gemische mit
a) einer bei Bestrahlung eine starke Säure bildenden Verbindung,
b) entweder einer Verbindung mit mindestens einer durch Säure spaltbaren C-O-C- oder C-O-Si-Bindung (für ein positiv arbeitendes Gemisch) oder einer Verbindung mit mindestens zwei durch Säure vernetzbaren Gruppen (für ein negativ arbeitendes Gemisch) und
c) einem in Wasser unlöslichen, in wäßrig-alkalischen Lösungen dagegen löslichen oder zumindest quellbaren Bindemittel.

Die Erfindung betrifft weiterhin daraus hergestellte strahlungsempfindliche Aufzeichnungsmaterialien, die zur Herstellung von Photoresists, elektronischen Bauteilen, Druckplatten oder zum Formteilätzen geeignet sind.

Strahlungsempfindliche Gemische mit Sulfonsäureestern als Säurebildnern eignen sich besonders für die Bestrahlung mit "Deep-UV"-Strahlung mit einer Wellenlänge von 220 bis 300 nm, insbesondere von 248 nm (bei dieser Wellenlänge emittieren KrF-Excimer-Laser). Als Sulfonsäureester wurden bisher Sulfonate von substituierten Phenolen (JP-A 03-223 864) oder von Hydroxymethylbenzoinen (DE-A 19 19 678), 2-Nitro-benzylsulfonate (EP-A 0 330 386), Pyrogallolsulfonate (T. Ueno et al. in "Polymers for Microelectronics - Science and Technology", Hrsg. Y. Tabata et al., Kodansha-Weinheim-New York, 1989, S. 66-67), N-Sulfonyloxy-imide (EP-A 0 388 343) oder 1-Sulfonyloxy-pyridone (DE-A 41 12 967) eingesetzt. Nachteile dieser Verbindungen sind eine zu hohe intrinsische Absorption im DUV-Bereich, eine nicht ausreichende Empfindlichkeit (durch eine zu geringe Quantenausbeute bei der säurebildenden Reaktion) oder eine zu geringe Löslichkeit. Dies führt zu Mängeln in der Qualität der Bildwiedergabe, zu niedrigen Durchsatzgeschwindigkeiten der zu strukturierenden Wafer oder zu Entmischungsproblemen.

Viele positiv arbeitende Photoresistschichten enthalten neben den säurebildenden aromatischen Sulfonsäureestern noch Verbindungen mit säurespaltbaren Gruppen. Werden solche Schichten bildmäßig bestrahlt, so ist zu beobachten, daß die Linienbreite, die man nach dem Entwickeln erhält, von der Lagerzeit zwischen Bestrahlung und Ausheizschritt (post-exposure bake) beeinflußt wird und damit Abweichungen von der Vorlage auftreten. Deshalb muß die Bestrahlungsdosis der Lagerzeit angepaßt werden. Diese Abhängigkeit der Bildqualität von der Lagerzeit stellt ein großes technisches Problem dar, dessen Lösung bislang noch nicht gelungen ist. Vermutlich sind dafür mehrere Ursachen verantwortlich. So wird angenommen, daß aus vielen der bisher eingesetzten säurebildenden Verbindungen Photolyseprodukte hervorgehen, die nicht stabil sind und während der Lagerzeit auf unerwünschte Weise reagieren. Daneben führen längere Lagerzeiten häufig zur Bildung von unlöslichen Deckschichten, deren verlängerte Entwicklungszeiten bei der weitgehenden Automatisierung der Halbleiterherstellung zu Komplikationen führen.

Aufgabe der Erfindung war es daher, ein strahlungsempfindliches Gemisch bereitzustellen, das eine hohe Empfindlichkeit insbesondere für DUV-Strahlung und gleichzeitig ein hohes Auflösungsvermögen bis in den Bereich von weniger als 500 nm besitzt. Es soll, unabhängig von der Zeit der Lagerung zwischen bildmäßigem Bestrahlen und nachfolgendem Erwärmen, einfach verarbeitbar sein und hohe Prozeßstabilität und -breite mit detailgenauer Reproduzierbarkeit und Zuverlässigkeit verbinden.

Gelöst wird die Aufgabe durch das Bereitstellen eines strahlungsempfindlichen, positiv oder negativ arbeitenden Gemisches mit
a) einer bei Bestrahlung eine starke Säure bildenden Verbindung,
b) entweder einer Verbindung mit mindestens einer durch Säure spaltbaren C-O-C- oder C-O-Si-Bindung (für ein positiv arbeitendes Gemisch) oder einer Verbindung mit mindestens zwei durch Säure vernetzbaren Gruppen (für ein negativ arbeitendes Gemisch) und
c) einem in Wasser unlöslichen, in wäßrig-alkalischen Lösungen dagegen löslichen oder zumindest quellbaren Bindemittel,
das dadurch gekennzeichnet ist, daß die Verbindung a) ein Sulfonsäureester der Formeln

[R¹-CR²(CF₃)-O-SO₂-]ₙR³ (I)

oder

R¹[-CR²(CF₃)-O-SO₂-R³)ₘ (II)

ist, worin
- R¹: in den Verbindungen der Formel I ein ein- oder mehrkerniger nichtkondensierter oder kondensierter (C₆-C₁₄)Aryl- oder (C₄-C₁₁)Heteroarylrest mit Sauerstoff, Schwefel oder Stickstoff als Heteroatom und in den Verbindungen der Formel II ein zwei- oder dreiwertiger Rest eines ein- oder mehrkernigen nichtkondensierten oder kondensierten (C₆-C₁₄)-Aromaten oder (C₄-C₁₂)Heteroaromaten mit Sauerstoff, Schwefel oder Stickstoff als Heteroatom oder ein zweiwertiger Rest der Formel -C₆H₄-X-C₆H₄-, worin X für ein Sauerstoffatom, eine Carbonyl- oder Sulfonylgruppe oder eine Gruppe CR⁷R⁸, in der R⁷ und R⁸ gleich oder verschieden sind und einen Methyl- oder Trifluormethylrest bedeuten,
- R²: ein Wasserstoffatom, ein Methyl-, Trifluormethyl- oder Cyanorest,
- R³: in den Verbindungen der Formel I mit n = 1 sowie in den Verbindungen der Formel II ein geradkettiger oder verzweigter, unsubstituierter oder substituierter (C₁-C₁₈)Alkylrest, ein (C₄-C₁₀)Cycloalkylrest, ein (C₂-C₆)Alkenylrest, ein unsubstituierter oder substituierter (C₆-C₁₄)Aryl- oder (C₄-C₁₂)-Heteroarylrest mit Sauerstoff, Schwefel oder Stickstoff als Heteroatom oder ein (C₇-C₁₈)Aralkylrest, in den Verbindungen der Formel I mit n = 2 oder 3 ein zwei- oder dreiwertiger Rest eines geradkettigen oder verzweigten, unsubstituierten oder substituierten (C₁-C₁₈)Alkans oder Cycloalkans, eines ein- oder mehrkernigen (C₆-C₁₄)Aromaten oder eines (C₄-C₁₂)-Heteroaromaten mit Sauerstoff, Schwefel oder Stickstoff als Heteroatom,
ist und
- n: eine ganze Zahl von 1 bis 3 und
- m: 2 oder 3 bedeutet.

Der Rest R¹ ist in den Verbindungen der Formel I bevorzugt ein Phenyl-, Naphthyl-, Thiophen-2- oder -3-yl, Benzo[b]thiophen-2- oder -3-yl, sowie ein Phenylrest, an den ein fünf- oder sechsgliedriger Ring mit 1 bis 3 Heteroatomen, wie Sauerstoff, Stickstoff oder Schwefel ankondensiert ist, vorzugsweise ein Chromanyl-, Chromenyl-, Thiochromanyl-, Isochromanyl- oder Isothiochromanyl-, Indolinyl-, Benzothiazolyl-, Chinolinyl-, 1,2,3,4-Tetrahydrochinolinyl- oder Isochinolinylrest. Er kann mit ein bis drei gleichartigen oder verschiedenen Substituenten, wie Fluor-, Chlor- oder Bromatomen, (C₁-C₆)Alkyl-, (C₂-C₆)Alkenyl-, (C₂-C₄)Alkinyl-, (C₁-C₆)Alkoxy-, (C₆-C₁₀)Aryl-, (C₆-C₁₀)Aryloxy-, (C₇-C₁₄)Aralkyl-, (C₇-C₁₄)Aryloxyalkyl-, (C₇-C₁₄)Aryloxyalkoxy-, (C₁-C₆)-Alkylthio-, (C₆-C₁₀)Arylthio-, Di(C₁-C₄)alkylamino-, (C₁-C₆)Carboxyalkyl-, Cyano-, Nitro-, (C₁-C₁₀)Alkansulfonyl-, (C₆-C₁₄)Arylsulfonylgruppen oder den Gruppen R³-SO₂-O-, R³-SO₂-NH-, HO-CR²(CF₃)- oder R³-SO₂-O-CR²(CF₃)- substituiert sein, wobei R² und R³ für die vorstehend beschriebenen einwertigen Reste steht.

Bevorzugte zweiwertige Reste R¹ sind meta- und para-Phenylen. Ein bevorzugter nicht-kondensierter zweiwertiger Rest ist Biphenyl-4,4'-diyl, ein bevorzugter kondensierter Naphthalin-1,4-diyl. Von den Resten der Formel -C₆H₄-X-C₆H₄- ist Oxy-biphenyl-4,4'-diyl bevorzugt. Die mehrwertigen Reste R¹ können in der gleichen Weise substituiert sein wie die einwertigen.

R² ist bevorzugt ein Trifluormethylrest.

In den Verbindungen der Formel I mit n = 1 sowie in den Verbindungen der Formeln II ist R³ bevorzugt ein (C₁-C₆)-Alkylrest, wie Methyl, Ethyl, Propyl, Isopropyl, oder Butyl, ein Cycloalkylrest, wie Cyclohexyl, ein per- oder hochfluorierten (C₁-C₆)Alkylrest, wie Trifluormethyl, ein (C₆-C₁₀)Arylrest, wie Phenyl, Tolyl, Naphthyl, Dihydro- oder Tetrahydronaphthyl, (C₇-C₁₄)Aralkylrest, wie Benzyl, oder ein (C₄-C₉)Heteroarylrest, wie Thiophen-2- oder -3-yl. In den Verbindungen der Formel II mit n = 2 ist R³ bevorzugt ein (C₁-C₁₀)Alkylen-, insbesondere Methylen, Ethan-1,2-diyl oder Propan-1,3-diyl, oder ein (C₆-C₁₀)-Arylen, insbesondere meta- oder para-Phenylen oder Naphthalindiyl. Die Reste R³ können in der gleichen Weise wie die Reste R¹ substituiert sein. Verbindungen, in denen der Rest R³ mehr als 15 Kohlenstoffatome enthält, liefern zwar Säuren, die noch weniger diffundieren, diese Verbindungen zeigen jedoch eine verminderte Strahlungsempfindlichkeit.

Die Verbindungen der Formel I und II lassen sich beispielsweise durch Umsetzen von aliphatischen oder aromatischen Sulfonsäurechloriden mit gegebenenfalls substituiertem l-Aryl-2,2,2-trifluor-ethanol oder 2-Aryl-1,1,1-trifluor-propan-2-ol herstellen. Die Umsetzung wird allgemein in einem Lösemittel, wie Tetrahydrofuran, Methylenchlorid oder vorzugsweise Dimethylformamid (DMF) in Gegenwart einer Base, wie Triethylamin, Pyridin oder vorzugsweise Natriumhydrid, durchgeführt. Die als Ausgangsmaterialien verwendeten fluorierten 1-Aryl-ethanole und 2-Aryl-propan-2-ole sind nach Verfahren erhältlich, die dem Fachmann allgemein bekannt sind. 2-Aryl-1,1,1,3,3,3-hexafluor-propan-2-ol läßt sich beispielsweise durch Umsetzung eines aktivierten Aromaten mit Hexafluoraceton herstellen. Weniger aktivierte Aromaten reagieren mit Hexafluoraceton in Gegenwart geringer Mengen von Lewissäure-Katalysatoren, vorzugsweise Aluminiumtrichlorid. Auch Grignard-Verbindungen, hergestellt beispielsweise aus bromierten oder jodierten Aromaten, lassen sich mit Hexafluoraceton zu den gewünschten Ausgangsmaterialien umsetzen.

Nur wenige Verbindungen der Formeln I und II sind bekannt. So offenbaren G.W. Astrologes und J.C. Martin (J. Amer. Chem. Soc. **99** [1977] 4400 - 4404) Verbindungen der oben angegebenen Formel I, n = 1, R¹ = Phenyl oder 4-tert.-Butyl-phenyl, R² = R³ = CF₃. D.W. Reynolds et al (J. Org. Chem. **55** [1990] 4448 - 4454) offenbaren eine Verbindung der Formel II, m = 2, R¹ = -C₆H₃(CH₃)-C(CF₃)₂-C₆H₃(CH₃)-, R² = CF₃ und R³ = CH₃. In dem Aufsatz von A.D. Allen et al. (J. Amer. Chem. Soc. **108** [1986] 3470 - 3474) sind weitere Verbindungen der Formel I mit n = 1, R¹ = Phenyl, 4-Fluor-phenyl, 4-Methoxy-phenyl oder p-Tolyl, R² = CF₃ und R³ = p-Tolyl sowie Verbindungen mit n = 1, R¹ = Phenyl oder p-Tolyl, R² = CN und R³ = p-Tolyl beschrieben. Untersucht wurde die Kinetik der Solvolyse dieser Verbindungen in verschiedenen Lösemitteln. Die Verbindungen der Formel I mit n = 2 oder 3 sowie die Verbindungen der Formel II, mit Ausnahme der einen bekannten Verbindung, sind dagegen neu und Teil der Erfindung. Auch die Verwendung von Verbindungen der Formel I oder II als Photosäuregeneratoren in einem strahlungsempfindlichen Gemisch ist neu und Teil der vorliegenden Erfindung. Überraschend war der Befund, daß die Strahlungsempfindlichkeit dieser Sulfonsäureester in der Regel um ein Vielfaches höher ist als das der bekannten Sulfonsäureester. Besonders überraschend war jedoch die Beobachtung, daß sich mit diesen Verbindungen eine strahlungsempfindliche Schicht herstellen läßt, in der das nach der bildmäßigen Bestrahlung entstandene latente Bild deutlich stabilisiert ist.

Die Reste R¹ und R³ in den Verbindungen der allgemeinen Formeln I und II beeinflussen die Effizienz der Säurebildung nur wenig, ein stark elektronegativer Substituent R² steigert dagegen die Effizienz. Daher sind auch Verbindungen, die den -C(CF₃)₂-O-SO₂-Rest als Strukturelement enthalten, besonders geeignete Säurebildner. Aus nicht geklärten Gründen sind solche Verbindungen, die zwei oder mehrere Reste dieses Typs enthalten besonders effiziente Säurebildner. Überraschenderweise besitzen gerade diese Verbindungen daneben auch eine gute thermische Stabilität und eine ausreichende Solvolysebeständigkeit, so daß die Verbindungen und die damit hergestellten Gemische längere Zeit gelagert werden können, ohne sich dabei zu verändern. Die mit den erfindungsgemäßen Gemischen, insbesondere den positiv arbeitenden, hergestellte Schichten zeigen deutlich bessere Eigenschaften als Schichten aus bekannten Gemischen. Unerwünschte Effekte, wie etwa die Änderung der Linienbreiten, der Empfindlichkeit oder der Abtragsraten bei unterschiedlichen Lagerzeiten zwischen Belichtung und dem nachfolgenden Ausheizschritt, treten deutlich weniger auf.

In dem erfindungsgemäßen Gemisch können auch verschiedene säurebildende Verbindungen der allgemeinen Formeln I und/oder II kombiniert sein.

Kombinationen mit bisher verwendeten photolytischen Säurespendern, beispielsweise Onium-Salzen wie Diazonium-, Phosphonium-, Sulfonium- und Jodonium-Salze von nicht nukleophilen Säuren, z.B. von HSbF₆, HAsF₆ oder HPF₆, Halogenverbindungen, insbesondere Trichlormethyltriazinderivaten oder Trichlormethyloxadiazolderivaten, o-Chinondiazidsulfonylchloriden oder Organometall-Organohalogen-Kombinationen sind ebenfalls möglich, aber die bereits erwähnten Nachteile können wieder verstärkt auftreten.

Der Anteil an Verbindungen der allgemeinen Formel I und/oder II im erfindungsgemäßen Gemisch liegt im allgemeinen bei 0,5 bis 25 Gew.-%, bevorzugt bei 1 bis 10 Gew.-%, jeweils bezogen auf das Gesamtgewicht der Feststoffe in dem Gemisch.

Neben den Photosäuregeneratoren der allgemeinen Formel I oder II enthalten die erfindungsgemäßen Gemische eine säureempfindliche Verbindung und ein filmbildendes Polymer. Das erfindungsgemäße Gemisch kann je nach Art der säureempfindlichen Verbindung positiv oder negativ arbeiten. Im Falle positiv arbeitender Materialien sind Gemische mit säurespaltbaren Löslichkeitsinhibitoren bevorzugt.

Als säurespaltbares Material in dem erfindungsgemäßen strahlungsempfindlichen Gemisch haben sich vor allem bewährt:
a) Verbindungen mit mindestens einer Orthocarbonsäureester- und/oder Carbonsäureamidacetalgruppe, wobei die Verbindungen auch polymeren Charakter haben und die genannten Gruppen als verknüpfende Elemente in der Hauptkette oder als seitenständige Substituenten auftreten können,
b) oligomere oder polymere Verbindungen mit wiederkehrenden Acetal- und/oder Ketalgruppen in der Hauptkette,
c) Verbindungen mit mindestens einer Enolether- oder N-Acyliminocarbonatgruppe,
d) cyclische Acetale oder Ketale von β-Ketoestern oder -amiden,
e) Verbindungen mit Silylethergruppen,
f) Verbindungen mit Silyl-enolethergruppen,
g) Monoacetale bzw. Monoketale, deren Aldehyd- bzw. Ketonkomponente eine Löslichkeit im Entwickler zwischen 0,1 und 100 g/l aufweist,
h) Ether auf der Basis tertiärer Alkohole,
i) Carbonsäureester und Carbonate, deren Alkoholkomponente ein tertiärer Alkohol, ein Allylalkohol oder ein Benzylalkohol ist, und
j) N,O-Acetale, insbesondere N,O-Polyacetale.

Es können auch Mischungen der genannten säurespaltbaren Materialien eingesetzt werden, was jedoch weniger bevorzugt ist. Von den genannten säurespaltbaren Materialien sind die mit C-O-C-Bindungen bevorzugt. Besonders bevorzugt sind Materialien, die den Typen (a), (b), (g), (i) und (j) angehören. Unter Typ (b) sind die polymeren Acetale hervorzuheben; von den säurespaltbaren Materialien des Typs (g) insbesondere diejenigen, deren Aldehyd- bzw. Ketonkomponente einen Siedepunkt von größer als 150 °C, vorzugsweise größer als 200 °C, aufweist. Ganz besonders bevorzugt sind jedoch die N,O-Acetale des Typs (j).

Der Anteil an säurespaltbaren Verbindungen in dem erfindungsgemäßen strahlungsempfindlichen Gemisch sollte bei 1 bis 50 Gew.-%, vorzugsweise bei 5 bis 40 Gew.-%, jeweils bezogen auf das Gesamtgewicht der Feststoffe, liegen.

Für ein negativ arbeitendes Gemisch kommen als säurevernetzbare Verbindungen Resole, sowie Aromaten, Heterocyclen und monomere oder oligomere Melamin- bzw. Harnstoff-Formaldehyd-Kondensate in Frage, die jeweils mehrfach hydroxymethyl-, alkoxymethyl-, insbesondere methoxymethyl-, acetoxymethyl- oder glycidylsubstituiert sind. Beispiele für geeignete Resole sind insbesondere die in Handel erhältlichen ^{(R)}Bakelite R 5363, R 17620 und R 10282 und ^{(R)}Kelrez 40-152. Resolderivate verursachen im tiefen UV-Bereich verhältnismäßig hohe Absorptionen, was zur Beeinträchtigung der Bildwiedergaben führen kann, und sind deshalb nicht bevorzugt.
Gut geeignet sind die bekannten Vernetzer der allgemeinen Formel III

(R⁶O-CHR⁵)ₒ-A-(CHR⁵-OR⁴)ₚ (III)

in der
- A: -B- oder -B-Y-B- ist und
- B: den zweiwertigen Rest eines unsubstituierten oder substituierten einkernigen aromatischen Kohlenwasserstoffs oder einer Sauerstoff oder Schwefel enthaltenden heterocyclischen aromatische Verbindung darstellt,
- Y: eine Einfachbindung, (C₁-C₄)Alkylen oder (C₁-C₄)Alkylendioxy, deren Ketten durch Sauerstoffatome unterbrochen sein können, -O-, -S-, -SO₂-, -CO-, -CO₂-, -O-CO₂-, -CO-NH- oder Phenylendioxy bedeutet,
- R⁴ und R⁶: Wasserstoff, (C₁-C₆)Alkyl, C₅- oder C₆-Cycloalkyl-, unsubstituiertes oder substituiertes (C₆-C₁₂)Aryl, (C₆-C₁₂)Aralkyl oder Acyl sind,
- R⁵: Wasserstoff, (C₁-C₄)Alkyl oder unsubstituiertes oder substituiertes Phenyl bedeutet,
- o: eine ganze Zahl von 1 bis 3 und
- p: eine ganze Zahl von 0 bis 3 ist, wobei die Summe aus
- o+p: mindestens 2 ist.

Schließlich können auch niedermolekulare oder oligomere Silanole als Vernetzer Verwendung finden. Beispiele hierfür sind etwa Dimethyl- oder Diphenylsilandiole, oder bereits vorkondensierte Oligomere mit diesen Einheiten.

Die Vernetzer sind in Lage, bei erhöhten Temperaturen unter dem Einfluß der photolytisch erzeugten Säure mit den nachstehend beschriebenen Polymeren zu vernetzen; sie sind dadurch gekennzeichnet, daß sie unter den vorstehend beschriebenen Bedingungen ein Carboniumkation bilden können.

Der Anteil an säurevernetzbaren Verbindungen in dem erfindungsgemäßen strahlungsempfindlichen Gemisch liegt bei 1 bis 50 Gew.-%, vorzugsweise bei 5 bis 40 Gew.-%, jeweils bezogen auf das Gesamtgewicht der festen Bestandteile des Gemischs.

Die erfindungsgemäßen Gemische enthalten ferner mindestens ein polymeres, in Wasser unlösliches, in wäßrigen alkalischen Lösungen aber lösliches, zumindest aber quellbares Bindemittel. Das Bindemittel zeichnet sich dadurch aus, daß es die übrigen Bestandteile der erfindungsgemäßen Gemische gut löst und insbesondere im Wellenlängenbereich von 190 bis 300 nm eine möglichst geringe Eigenabsorption, d.h. eine hohe Transparenz, aufweist.

Novolak-Kondensationsharze können daher wegen ihrer hohen Eigenabsorption im genannten Wellenlängenbereich nur zusammen mit anderen, höher transparenten, Bindemitteln eingesetzt werden. Die Mischungsverhältnisse richten sich dabei vorwiegend nach der Art des mit dem Novolakharz zu mischenden Bindemittels. Im allgemeinen kann das Bindemittel des erfindungsgemäßen strahlungsempfindlichen Gemisches bis zu 30 Gew.-%, insbesondere bis zu 20 Gew.-%, eines Novolak-Kondensationsharzes enthalten.

Als Bindemittel geeignet sind Homo- oder Copolymere des 4-Hydroxystyrols sowie seiner Alkylderivate, z.B. des 3-Methyl-4-hydroxystyrols, des 2,3- oder 3,5-Dimethyl-4-hydroxystyrols sowie Homo- oder Copolymere anderer Polyvinylphenole, z. B. des 3-Hydroxystyrols oder Acrylate oder Acrylamide, die phenolische Hydroxygruppen aufweisen. Als Comonomere im Copolymeren können polymerisierbare Verbindungen wie Styrol, Methacrylsäuremethacrylat, Acrylsäuremethacrylat oder ähnliche eingesetzt werden.

Gemische mit erhöhter Plasmabeständigkeit werden dann erhalten, wenn zur Herstellung von Copolymeren des obigen Typs Silicium enthaltende Vinylmonomere, z. B. Vinyltrimethylsilan, verwendet werden. Die Transparenz dieser Bindemittel ist im interessierenden Bereich im allgemeinen sogar höher, so dap eine verbesserte Strukturierung möglich ist.

Mit gleichem Erfolg lassen sich auch Homo- oder Copolymere des Maleimids verwenden. Auch diese Bindemittel zeigen hohe Transparenz im beschriebenen Wellenlängenbereich. Als Comonomere werden auch hier bevorzugt Styrol, substituierte Styrole, Vinylether, Vinylester, Vinylsilanylverbindungen oder (Meth-)acrylsäureester eingesetzt.

Schließlich sind darüber hinaus auch Copolymere des Styrols mit Comonomeren verwendbar, die in wäprig alkalischen Lösungen eine Löslichkeitserhöhung bewirken. Hierzu zählen beispielsweise Maleinsäureanhydrid oder Maleinsäurehalbester.

Vorausgesetzt, daß die optischen Qualitäten der erfindungsgemäßen Gemische nicht verschlechtert werden, sind Kombinationen der genannten Bindemittel im erfindungsgemäßen Gemisch möglich, jedoch nicht bevorzugt.

Die Extinktion des Bindemittels bzw. der Kombination von Bindemitteln für Strahlung der Wellenlänge 248 nm beträgt bevorzugt weniger als 0,35 µm⁻¹, besonders bevorzugt weniger als 0,25 µm⁻¹.

Die Glasübergangstemperatur des Bindemittels bzw. der Kombination von Bindemitteln liegt vorteilhaft bei mindestens 120 °C.

Der Anteil des Bindemittels beträgt im allgemeinen 1 bis 95 Gew.-%, vorzugsweise 5 bis 90 Gew.-%, besonders bevorzugt 30 bis 85 Gew.-%, jeweils bezogen auf das Gesamtgewicht der festen Anteile des strahlungsempfindlichen Gemischs.

Um speziellen Erfordernissen, wie Flexibilität, Haftung und Glanz zu genügen, können den erfindungsgemäßen strahlungsempfindlichen Gemischen auch noch Substanzen wie Polyglykole, Cellulose-Derivate wie Ethylcellulose, Farbstoffe, Pigmente, Weichmacher, Netzmittel und Verlaufmittel zugesetzt sein.

Gegenstand der vorliegenden Erfindungs ist schließlich auch ein Aufzeichnungsmaterial mit einem Träger und einer strahlungsempfindlichen Schicht aus dem erfindungsgemäßen Gemisch. Das Aufzeichnungsmaterial wird üblicherweise durch Beschichten des Trägers mit einer Lösung dieses Gemisches hergestellt.

Als Lösemittel dafür eignen sich besonders Ethylenglykol, Glykolether (wie Glykolmonomethylether, Glykoldimethylether und Glykolmonoethylether), Propylenglykolmonoalkylether (wie Propylenglykolmonomethylether), aliphatische Ester (wie Ethylacetat, Hydroxyethylacetat, Alkoxyethyl-acetat, n-Butylacetat und Amylacetat), Propylenglykol-monoalkyletheracetate (wie Propylenglykolmethyletheracetat), Ether (wie Tetrahydrofuran und Dioxan), Ketone (wie Methylethylketon, Methylisobutyl-keton, Cyclopentanon und Cyclohexanon), DMF, Dimethylacetamid, Hexamethylphosphorsäuretriamid, N-Methylpyrrolidon und Butyrolacton sowie beliebige Mischungen davon. Besonders bevorzugt sind Glykolether, aliphatische Ester und Ketone.

Letztendlich hängt die Wahl des Lösemittels bzw. Lösemittelgemisches von dem angewendeten Beschichtungsverfahren, der gewünschten Schichtstärke und den Trocknungsbedingungen ab. Ebenso müssen die Lösemittel chemisch neutral sein, d. h. sie dürfen nicht mit den übrigen Schichtkomponenten irreversibel reagieren.

Die zum Beschichten verwendeten Lösungen haben in der Regel einen Feststoffgehalt von 5 bis 60 Gew.-%, vorzugsweise 10 bis 50 Gew.-%.

Als Träger kommen alle Materialien in Frage, aus denen Kondensatoren, Halbleiter, mehrlagige gedruckte Schaltungen oder integrierte Schaltkreise bestehen oder hergestellt werden können. Insbesondere sind Oberflächen aus thermisch oxidiertem und/oder mit Aluminium beschichtetem Siliciummaterial zu nennen, die auch dotiert sein können, einschließlich aller anderen in der Halbleitertechnologie üblichen Substrate, wie beispielsweise Siliciumnitrid, Galliumarsenid und Indiumphosphid. Weiterhin kommen die aus der Flüssigkristalldisplay-Herstellung bekannten Substrate in Frage, wie Glas und Indium-Zinnoxid, ferner Metallplatten und -folien (beispielsweise aus Aluminium, Kupfer, Zink), Bimetall- und Trimetallfolien, aber auch elektrisch nicht leitende Folien, die mit Metallen bedampft sind, ggf. mit Aluminium beschichtete SiO₂-Materialien und Papier. Diese Substrate können einer Temperaturvorbehandlung unterzogen werden, oberflächlich aufgerauht, angeätzt oder zur Verbesserung erwünschter Eigenschaften, z. B. der Erhöhung der Hydrophilie, mit Chemikalien behandelt sein.

Um eine bessere Haftung der Resistschicht auf dem Substrat zu erreichen, kann das Gemisch einen Haftvermittler enthalten. Die Substratoberfläche kann auch mit einem Haftvermittler vorbehandelt sein. Bei Silicium- bzw. Siliciumdioxid-Substraten kommen hierfür Haftvermittler vom Aminosilan-Typ, wie 3-Aminopropyltriethoxysilan oder Hexamethyl-disilazan, in Frage.

Geeignete Trägermaterialien für die Herstellung von Druckformen (Hoch-, Flach-, Siebdruck und Reliefkopien) sind Aluminiumplatten (ggf. anodisch oxidiert, gekörnt und/oder silikatisiert), Zink- und Stahlplatten (ggf. verchromt) sowie Kunststoffolien oder Papier.

Das erfindungsgemäpe Aufzeichnungsmaterial wird bild-mäßig belichtet. Quellen aktinischer Strahlung sind: Metallhalogenidlampen, Kohlebogenlampen, Xenonlampen und Quecksilberdampflampen. Ebenso kann eine Belichtung mit energiereicher Strahlung wie Laser-, Elektronen- oder Röntgenstrahlung erfolgen. Besonders bevorzugt sind jedoch Lampen, die Licht einer Wellenlänge von 190 bis 260 nm ausstrahlen können, d.h. insbesondere Xenon- oder/und Quecksilberdampflampen. Darüber hinaus lassen sich auch Laserlichtquellen verwenden, z. B. Excimerlaser, insbesondere KrF- oder ArF-Laser, die bei 248 bzw. 193 nm emittieren. Die Strahlungsquellen müssen in den genannten Wellenlängenbereichen eine ausreichende Emission aufweisen.

Die Schichtstärke variiert in Abhängigkeit von ihrem Einsatzgebiet. Sie beträgt zwischen 0,1 und 100 µm, insbesondere zwischen 1 und 10 µm.

Das Auftragen des strahlungsempfindlichen Gemisches auf das Substrat kann durch Aufsprühen, Fließbeschichten, Walzen, Schleuder- und Tauchbeschichten erfolgen. Danach wird das Lösemittel durch Verdampfen entfernt, so daß auf der Oberfläche des Substrats die strahlungsempfindliche Schicht zurückbleibt. Die Entfernung des Lösemittels kann ggf. durch Erhitzen der Schicht auf Temperaturen bis zu 150 °C gefördert werden. Das Gemisch kann aber auch zunächst auf obengenannte Weise auf einen Zwischenträger aufgetragen werden, von dem aus es unter Druck und erhöhter Temperatur auf das endgültige Trägermaterial übertragen wird. Als Zwischenträger können grundsätzlich alle auch als Trägermaterialien ausgewiesene Materialien Anwendung finden. Anschließend wird die Schicht bildmäßig bestrahlt. Danach wird in der Schicht durch Entwickeln ein Bildmuster freigelegt, indem die Schicht mit einer Entwicklerlösung behandelt wird, die die bestrahlten Bereiche des Materials löst bzw. entfernt.

Als Entwickler werden insbesondere wäßrige Lösungen von Silikaten, Metasilikaten, Hydroxiden, Hydrogen- oder Dihydrogenphosphaten, Carbonaten oder Hydrogencarbonaten verwendet. Metallionenfreie Entwickler sind bevorzugt. Der Gehalt dieser Substanzen in der Entwicklerlösung beträgt im allgemeinen 0,1 bis 15 Gew.-%, vorzugweise 0,5 bis 5 Gew.-%, bezogen auf das Gewicht der Entwicklerlösung.

Der Entwickler kann geringe Mengen eines Netzmittels enthalten, um die Ablösung der belichteten Stellen im Entwickler zu erleichtern.

Die entwickelten Resiststrukturen werden ggf. nachgehärtet. Dies geschieht im allgemeinen dadurch, daß man die Resiststruktur auf einer hot plate bis zu einer Temperatur unterhalb der Fließtemperatur erhitzt und anschließend mit UV-Licht einer Xenon-Quecksilber-Dampflampe (Bereich von 200 bis 250 nm) ganzflächig belichtet. Durch diese Nachhärtung werden die Resiststrukturen vernetzt, so daß die Strukturen eine Fließbeständigkeit im allgemeinen bis zu Temperaturen von über 200 °C aufweisen. Die Nachhärtung kann auch ohne Temperaturerhöhung durch Einstrahlen von energiereichem UV-Licht erfolgen.

Bevorzugte Anwendung findet das erfindungsgemäße Gemisch in lithographischen Prozessen zur Herstellung integrierter Schaltungen oder von diskreten elektrischen Bausteinen. Das aus dem Gemisch hergestellte Aufzeichnungsmaterial dient dabei als Maske für die folgenden Prozeßschritte. Hierunter zählen z. B. das Ätzen des Schichtträgers, die Implantation von Ionen in den Schichtträger oder die Abscheidung von Metallen oder anderen Materialien auf den Schichtträger.

### Herstellungsbeispiel 1:

Zu einer Suspension von 1,05 g Natriumhydrid (80%ige Dispersion in Mineralöl; 35 mmol) in 100 ml Diethylether wurde eine Lösung aus 5 g (30 mmol) 1-Phenyl-2,2,2-trifluor-ethanol in 100 ml Diethylether zugefügt. Nach 1-stündigem Rühren bei Raumtemperatur wurden 5,7 g (30 mmol) Toluol-4-sulfonylchlorid, gelöst in 100 ml Diethylether, zugetropft. Die Mischung wurde 8 Stunden bei Raumtemperatur gerührt und über Nacht stehengelassen. Nach Filtration des Salzes wurde die organische Phase mit Wasser ausgeschüttelt und getrocknet. Nach Einengen und Umkristallisieren des Rückstands aus Petrolether/Diethylether verblieben 7,2 g 2,2,2-Trifluor-1-phenyl-1-(toluol-4-sulfonyloxy)-ethan (farblose Kristalle, Schmelzpunkt (Smp.): 115 °C).

l-Phenyl-2,2,2-trifluor-ethanol wurde durch Reduktion von Phenyl-trifluormethyl-keton mit Natriumborhydrid erhalten.

### Herstellungsbeispiel 2:

Zu 5 g (26 mmol) 1,1,1-Trifluor-2-phenyl-propan-2-ol in 125 ml Diethylether wurden 0,9 g (30 mmol) Natriumhydrid (80%ige Dispersion in Mineralöl) bei Raumtemperatur zugegeben. Die Mischung wurde etwa 1 Stunde gerührt, und dann portionsweise mit 4,75 g (25 mmol) Toluol4-sulfonylchlorid versetzt. Nach 60 Stunden Rühren bei Raumtemperatur wurden die ungelösten Bestandteilen abfiltriert und die Lösung anschließend gekühlt. Dabei fielen 4,3 g 1,1,1-Trifluor-2-phenyl-2-(toluol-4-sulfonyloxy)-propan (farblose Kristalle, Smp.: 102 °C [aus Diethylether umkristallisiert]) an. Nach Einengen der Mutterlauge konnten weitere 1,6 g der gewünschten Verbindung isoliert werden.

1,1,1-Trifluor-2-phenyl-propan-2-ol wurde durch AlCl₃-katalysierte Friedel-Crafts-Reaktion aus Benzol und 1,1,1-Trifluor-aceton hergestellt.

### Herstellungsbeispiel 3:

5,2 g (25 mmol) 1-Cyano-2,2,2-trifluor-1-phenyl-ethanol wurden in 50 ml DMF gelöst und mit 0,9 g (30 mmol) Natriumhydrid (80%ige Dispersion in Mineralöl) versetzt. Zu der praktisch klaren Lösung wurden nach etwa 1 Stunde 5,5 g (29 mmol) Toluol-4-sulfonylchlorid portionsweise hinzugefügt. Die Mischung wurde 8 Stunden gerührt, über Nacht stehengelassen, und dann in Wasser gegossen. Die sich abscheidende ölige Flüssigkeit erstarrte beim Kühlen. Der Feststoff wurde abgesaugt und getrocknet. Man erhielt 6,3 g 1-Cyano-1-phenyl-1-(toluol-4-sulfonyloxy)-2,2,2-trifluor-ethan (farblose Kristalle, Smp.: 70 °C [aus Ligroin]) erwiesen.

1-Cyano-1-phenyl-2,2,2-trifluor-ethanol wurde aus α,α,α-Trifluoracetophenon durch Cyanhydrin-Reaktion mit Kaliumcyanid hergestellt.

### Herstellungsbeispiel 4:

5,16 g (20 mmol) 1,1,1,3,3,3-Hexafluor-2-p-tolyl-propan-2-ol wurden in 50 ml Methylenchlorid gelöst und mit 2, 8 g (28 mmol) Triethylamin versetzt. Zu der auf 5 °C gekühlten Lösung wurden tropfenweise unter Rühren 3,2 g (22 mmol) Methansulfonylchlorid zugetropft. Nach Erwärmen auf Raumtemperatur wurde die Mischung über Nacht stehengelassen. Das ausgefallene Salz wurde abfiltriert und die organische Lösung eingeengt. Die anschließende Chromatographie (Kieselgel/Methylenchlorid) ergab 4,8 g 2-Methansulfonyloxy-2-p-tolyl-1,1,1,3,3,3-hexafluor-propan (hellgelbes Öl).

1,1,1,3,3,3-Hexafluor-2-p-tolyl-propan-2-ol wurde durch AlCl₃-katalysierte Friedel-Crafts-Reaktion aus Toluol und Hexafluoraceton hergestellt.

### Herstellungsbeispiel 5:

12 g (44,4 mmol) 1,1,1,3,3,3-Hexafluor-2-(3-vinyl-phenyl)-propan-2-ol wurden in 50 ml wasserfreiem DMF gelöst und zur Stabilisierung mit einer Spatelspitze 2-tert.-Butyl-hydrochinon (10 mg) versetzt. Dann wurden unter Rühren und Eiskühlung bei ca. 5 °C portionsweise 1,5 g (50 mmol) Natriumhydrid hinzugefügt. Nachdem sich alles gelöst hatte, wurden bei der gleichen Temperatur 9,92 g (47 mmol) 4-Chlor-benzolsulfonylchlorid zugefügt. Die Mischung wurde 72 Stunden bei Raumtemperatur gerührt, anschliepend mit Methylenchlorid versetzt, mit Wasser gewaschen, getrocknet, filtriert und eingeengt. Der erhaltene Rückstand wurde mit Hexan/Chloroform auf einer Kieselgelsäule chromatographiert. Man isolierte 16,65 g 2-(4-Chlor-benzolsulfonyloxy)-1,1,1,3,3,3-hexafluor-2-(3-vinyl-phenyl)-propan (= 4-Chlor-benzolsulfonsäure-(2,2,2-trifluor-1-trifluormethyl-1-(3-vinyl-phenyl)-ethylester)) (farblose Kristalle, Smp.: 70 °C).

Das 1,1,1,3,3,3-Hexafluor-2-(3-vinyl-phenyl)-propan-2-ol wurde aus 3-Brom-styrol durch Umsetzung mit (i) Magnesium und (ii) Hexafluoraceton erhalten.

### Herstellungsbeispiel 6:

4,1 g (10 mmol) 1,3-Bis-(1-hydroxy-1-trifluormethyl-2,2,2-trifluor-ethyl)-benzol wurden in 50 ml Methylenchlorid gelöst und mit 2,5 g (25 mmol) Triethylamin versetzt. Dann wurden 3,64 g (25 mmol) Methansulfonylchlorid zugetropft. Die Mischung wurde 8 Stunden bei Raumtemperatur gerührt, vom ausgefallenen Salz abgetrennt, und eingeengt. Man erhielt 5,6 g 1,3-Bis-(1-methansulfonyloxy-1-trifluormethyl-2,2,2-trifluor-ethyl)-benzol (farblose Kristalle, Smp.: 125 °C [aus Diisopropylether]).

### Herstellungsbeispiel 7:

10,1 g (15,2 mmol) 1,1,1,3,3,3-Hexafluor-2,2-bis-[4-methyl-3-(2,2,2-trifluor-1-hydroxy-1-trifluormethyl-ethyl)-phenyl]-propan wurden in 50 ml Methylenchlorid gelöst und bei 0°C mit 3,4 g (33 mmol) Triethylamin versetzt. Dazu wurden bei 0 bis 5°C 4,0 g (34,7 mmol) Methansulfonylchlorid in 20 ml Methylenchlorid getropft. Die Mischung wurde 24 Stunden bei Raumtemperatur gerührt, vom ausgefallenen Salz abgetrennt, mit Wasser gewaschen, getrocknet und eingeengt. Der ölige Rückstand wurde umkristallisiert. Man erhielt 9,2 g 1,1,1,3,3,3-Hexafluor-2,2-bis-[4-methyl-3-(2,2,2-trifluor-1-methansulfonyloxy-1-trifluormethyl-ethyl)-phenyl]-propan (farblose Kristalle, Smp.: 135 °C [aus Toluol]).

Die Ausgangsverbindung wurde aus 1,1,1,3,3,3-Hexafluor-2,2-di-p-tolyl-propan durch Umsetzung mit (i) Brom, (ii) Magnesium und (iii) Hexafluoraceton hergestellt.

### Herstellungsbeispiel 8:

4,1 g (10 mmol) 1,4-Bis-(2,2,2-trifluor-1-hydroxy-1-trifluormethyl-ethyl)-benzol wurden in 50 ml Methylenchlorid gelöst und mit 2,5 g (25 mmol) Triethylamin versetzt. Dazu wurden bei 5 bis 10°C 2,86 g (25 mmol) Methansulfonylchlorid getropft. Die Mischung wurde 48 Stunden bei Raumtemperatur gerührt, vom ausgefallenen Salz abgetrennt, mit Wasser gewaschen, getrocknet und eingeengt. Der Rückstand wurde an einer Kieselgelsäule mit Methylenchlorid/n-Hexan (1:1) chromatographiert. Man erhielt 3,2 g 1,4-Bis-(2,2,2-trifluor-1-methansulfonyloxy-1-trifluormethyl-ethyl)-benzol (farblose Kristalle, Smp.: 151 °C [aus Diisopropylether]).

Als Nebenprodukt wurden 1,4 g 1-(2,2,2-Trifluor-1-methansulfonyloxy-1-trifluormethyl-ethyl)-4-(2,2,2-trifluor-1-hydroxy-1-trifluormethyl-ethyl)-benzol isoliert (farblose Kristalle, Smp.: 160°C).

### Herstellungsbeispiel 9:

4,25 g (10 mmol) 2,4-Bis-(2,2,2-trifluor-1-hydroxy-1-trifluormethyl-ethyl)-toluol wurden in 50 ml Methylenchlorid gelöst und mit 2,5 g (25 mmol) Triethylamin versetzt. Dazu wurden bei 5 bis 10°C 2,86g (25 mmol) Methansulfonylchlorid getropft. Die Mischung wurde 16 Stunden bei Raumtemperatur gerührt, vom ausgefallenen Salz abgetrennt, mit wasser gewaschen, getrocknet und eingeengt. Der Rückstand wurde an einer Kieselgelsäule mit Methylenchlorid/n-Hexan (1:1) chromatographiert. Nach Einengen des Eluats erhielt man 2,9 g 2,4-Bis-(2,2,2-trifluor-1-methansulfonyloxy-1-trifluormethyl-ethyl)-toluol (farblose Kristalle, Smp.: 107 °C).

### Herstellungsbeispiel 10:

4,1 g (10 mmol) 1,3-Bis-(2,2,2-trifluor-1-hydroxy-1-trifluormethyl-ethyl)-benzol wurden in 20 ml trockenem DMF gelöst und mit 0,75 g (25 mmol) Natriumhydrid (80%ige Dispersion) versetzt. Die entstandene klare Lösung wurde 30 min nachgerührt. Dann wurden bei 10 bis 15°C 3,22 g (25 mmol) Ethansulfonsäurechlorid zugetropft. Die Mischung wurde 48 Stunden bei Raumtemperatur gerührt, vom ausgefallenen Salz abgetrennt, mit Wasser gewaschen, getrocknet und eingeengt. Der Rückstand wurde an einer Kieselgelsäule mit Methylenchlorid/n-Hexan (1:1) eluiert. Man erhielt 3,5 g 1,3-Bis-(1-ethansulfonyloxy-2,2,2-trifluor-1-trifluormethyl-ethyl)-benzol (farblose Kristalle, Smp.: 67°C).

Als Nebenprodukt wurden 1,2 g 1-(1-Ethansulfonyloxy-2,2,2-trifluor-1-trifluormethyl-ethyl)-3-(2,2,2-trifluor-1-hydroxy-1-trifluormethyl-ethyl)-benzol isoliert (farblose Kristalle, Smp.: 64°C).

### Herstellungsbeispiel 11:

4,27 g (10 mmol) 2,4-Bis-(2,2,2-trifluor-1-hydroxy-1-trifluormethyl-ethyl)-phenol wurden in 40 ml trockenem DMF gelöst und mit 1,05 g (35 mmol) Natriumhydrid (80%ige Dispersion) versetzt. Die entstandene klare Lösung wurde 30 min nachgerührt. Dazu wurden bei 5°C 4,0 g (35 mmol) Methansulfonylchlorid getropft. Die Mischung wurde 48 Stunden bei Raumtemperatur gerührt und in Eiswasser gegossen. Der Niederschlag wurde abgetrennt, mit einer wäßrigen Ethanollösung gewaschen und getrocknet. Der Rückstand wurde auf einer Kieselgelsäule mit Methylenchlorid chromatographiert. Es wurden 2,2 g 1,3-Bis-(2,2,2-trifluor-1-methansulfonyloxy-1-trifluormethylethyl)-4-methansulfonyloxy-benzol (farblose Kristalle, Smp.: 149°C) erhalten.

### Herstellungsbeispiel 12:

4,1 g (10 mmol) 1,3-Bis-(2,2,2-trifluor-1-hydroxy-1-trifluormethyl-ethyl)-benzol wurden in 20 ml trockenem DMF gelöst und mit 0,75 g (25 mmol) Natriumhydrid (80%ige Dispersion) versetzt. Die entstandene klare Lösung wurde 30 min nachgerührt. Dazu wurden bei 10 bis 15°C 4,75 g (25 mmol) Toluol-4-sulfonylchlorid in 15 ml DMF getropft. Die Mischung wurde 48 Stunden bei Raumtemperatur gerührt und in Wasser gegossen. Der kristalline Niederschlag wurde abfiltriert, mit Wasser gewaschen und getrocknet. Der Rückstand wurde an einer Kieselgelsäule mit Methylenchlorid chromatographiert. Man erhielt 3,5 g 1,3-Bis-[2,2,2-trifluor-1-(toluol-4-sulfonyloxy)-1-trifluormethyl-ethyl]-benzol (farblose Kristalle, Smp.: 131°C).

### Herstellungsbeispiel 13:

4,1 g (10 mmol) 1,3-Bis-(2,2,2-trifluor-1-hydroxy-1-trifluormethyl-ethyl)-benzol wurden in 20 ml trockenem DMF gelöst und mit 0,75 g (25 mmol) Natriumhydrid (80%ige Dispersion) versetzt. Die entstandene klare Lösung wurde 30 min nachgerührt. Dann wurden bei 10 bis 15°C 5,25 g (25 mmol) 4-Chlor-benzolsulfonylchlorid in 15 ml DMF zugetropft. Die Mischung wurde 48 Stunden bei Raumtemperatur gerührt und in Wasser gegossen. Der kristalline Niederschlag wurde abfiltriert, mit Wasser nachgewaschen und getrocknet. Der Rückstand wurde an einer Kieselgelsäule mit Methylenchlorid chromatographiert. Man erhielt 4,2 g 1,3-Bis-[1-(4-chlor-benzolsulfonyloxy)-2,2,2-trifluor-1-trifluormethyl-ethyl]-benzol (farblose Kristalle, Smp.: 125).

### Herstellungsbeispiel 14:

7,75 g (30 mmol) 1,1,1,3,3,3-Hexafluor-2-p-tolyl-propan-2-ol wurden in 45 ml DMF gelöst und mit 1,2 g (40 mmol) NaH (80%ige Dispersion in Mineralöl) versetzt. Nachdem eine klare Lösung entstanden war wurden 5,0 g (14 mmol) 2,4,6-Trimethyl-benzol-1,3-disulfonylchlorid hinzugefügt und die Mischung 72 Stunden bei Raumtemperatur nachgerührt. Das Gemisch wurde in Wasser gegossen, und das ausgefallene Produkt abfiltriert und getrocknet. Man erhielt 7,8 g 1,3,5-Trimethyl-2,4-bis-(2,2,2-trifluor-1-trifluormethyl-ethoxysulfonyl)-benzol (farblose Kristalle, Smp.: 178 °C [aus Acetonitril]).

### Herstellungsbeispiel 14A:

4,5 g (7,88 mmol) 1,3,5-Tris-(2,2,2-trifluor-1-hydroxy-1-trifluormethyl-ethyl)benzol wurden in 40 ml trockenem DMF gelöst und unter Stickstoffspülung mit 0,95 g (31,5 mmol) Natriumhydrid versetzt. Nach Auflösung des Hydrids wurden bei 0 bis 5 °C 6,56 g (30 mmol) 4-Isopropylbenzolsulfonylchlorid hinzugefügt. Es wurde eine leichte Wärmetönung beobachtet. Die Mischung wurde 20 Stunden bei 5 °C gerührt, mit Ethylacetat versetzt, mit Wasser gewaschen, getrocknet, filtriert und eingeengt. Man erhielt 9,6 g Rohprodukt, das über eine Kieselgelsäule mit einer Mischung aus Methylenchlorid/Hexan 1:1 eluiert wurde. Die dabei erhaltene dritte Fraktion wurde vom Lösemittel befreit und ergab 6,8 g 1,3,5-Tris-[2,2,2-trifluor-1-(4-isopropyl-benzolsulfonyloxy)-1-trifluormethyl-ethyl]-benzol. Fp. 105 °C.

### Herstellungsbeispiele 15 bis 209:

Die nachstehend in den Tabellen 1 und 1A aufgeführten Verbindungen der allgemeinen Formel I mit n = 1 wurden entsprechend den Herstellungsbeispielen 1 bis 5 hergestellt, wobei die Zahl in der letzten Spalte der Tabelle das jeweils analoge Beispiel angibt. In Tabelle 2 sind Verbindungen der allgemeinen Formel II wiedergegeben, in denen fluorierte Bis-(2-hydroxy-propan)-derivate entsprechend den Herstellungsbeispielen 6 bis 13 mit der zwei- oder dreifachen molaren Menge an Sulfonsäurechlorid verestert wurden. Tabelle 3 gibt Beispiele für Verbindungen der allgemeinen Formeln I mit n = 2 oder 3 an, in denen ein mehrfunktionelles Sulfonsäurechlorid entsprechend Herstellungsbeispiel 14 mit fluorierten 2-Hydroxy-propan-derivaten verestert wurde.

**Tabelle 1**

| | | | | |
|---|---|---|---|---|
| Verbindungen der allgemeinen Formel I R¹-C(CF₃) (R²)-O-SO₂-R³ | | | | |

| Nr. | R¹ | R² | R³ | gemäß Bsp. |
|---|---|---|---|---|
| 15 | -C₆H₅ | -H | -C₆H₄-Cl(p) | 1 |
| 16 | -C₆H₄-CH₃(p) | -H | -C₆H₄-CH₃(p) | 1 |
| 17 | -C₆H₄-OCH₃(p) | -H | -C₆H₄-CH₃(p) | 1 |
| 18 | -C₆H₄Br(p)- | -H | -C₆H₄-CH₃(p) | 1 |
| 19 | -C₆H₄-CF₃(m) | -H | -C₆H₄-CH₃(p) | 1 |
| 20 | -C₆H₅ | -CH₃ | -C₆H₄-Cl(p) | 2 |
| 21 | -C₆H₄-CH₃(p) | -CH₃ | -C₆H₄-CH₃(p) | 2 |
| 22 | -C₆H₄-CH₃(m) | -CH₃ | -C₆H₄-CH₃(p) | 2 |
| 23 | -C₆H₄-C₆H₅(p) | -CH₃ | -C₆H₄-CH₃(p) | 2 |
| 24 | -C₆H₄Cl(p)- | -CH₃ | -C₆H₄-CH₃(p) | 2 |
| 25 | -C₆H₄Cl(m)- | -CH₃ | -C₆H₄-CH₃(p) | 2 |
| 26 | -C₆H₄-CF₃(p) | -CH₃ | -C₆H₄-CH₃(p) | 2 |
| 27 | -C₆H₄-CF₃(m) | -CH₃ | -C₆H₄-CH₃(p) | 2 |
| 28 | -C₆H₅ | -CN | -C₆H₄-CH₃(p) | 3 |
| 29 | -C₆H₄-CH₃(p) | -CN | -C₆H₄-CH₃(p) | 3 |

**Tabelle 1A**

| | | | |
|---|---|---|---|
| Verbindungen der allgemeinen Formel I, in denen R² = -CF₃ ist | | | |

| Nr. | R¹ | R³ | gemäß Bsp. |
|---|---|---|---|
| 30 | -C₆H₅ | -CH₃ | 4 |
| 31 | -C₆H₅ | -CF₃ | * |
| 32 | -C₆H₅ | -CH₂-C₂H₅ | 5 |
| 33 | -C₆H₅ | -CH₂-C₆H₅ | 5 |
| 34 | -C₆H₅ | -C₆H₄-CH₃(p) | 5 |
| 35 | -C₆H₅ | -C₆H₄Cl(p) | 5 |
| 36 | -C₆H₅ | -C₆H₄-NO₂(p) | 5 |
| 37 | -C₆H₅ | -C₆H₄-CO₂CH₃(p) | 5 |
| 38 | -C₆H₅ | -C₆H₄-CO₂CH₃(m) | 5 |
| 39 | -C₆H₅ | -C₆H₄-OBuⁿ(p) | 5 |
| 40 | -C₆H₅ | -C₆H₄-OCF₃(p) | 5 |
| 41 | -C₆H₅ | -β-C₁₀H₇ | 5 |
| 42 | -C₆H₅ | 5-Cl-thiophen-2-yl | 4 |
| 43 | -C₆H₅ | 4-C₆H₅-SO₂-thiophen-2-yl | 5 |
| 44 | -C₆H₄-CH₃(p) | -C₂H₅ | 4 |
| 45 | -C₆H₄-CH₃(p) | -C₄H₉ | 5 |
| 46 | -C₆H₄-CH₃(p) | -C₁₂H₂₅ | 5 |
| 47 | -C₆H₄-CH₃(p) | -C₆H₄-CH₃(p) | 5 |
| 48 | -C₆H₄-CH₃(p) | -C₆H₄Cl(p)-CH₃(o) | 5 |
| 49 | -C₆H₄-CH₃(p) | -C₆H₄Cl(p) | 5 |
| 50 | -C₆H₄-CH₃(p) | -C₆H₃F₂(2,4)- | 5 |
| 51 | -C₆H₄-CH₃(p) | -C₆H₃Cl₂(2,4)- | 5 |
| 52 | -C₆H₄-CH₃(p) | -C₆H₃Cl₂(3,4)- | 5 |
| 53 | -C₆H₄-CH₃(p) | -C₆H₃Cl₂(2,5)- | 5 |
| 54 | -C₆H₄-CH₃(p) | -C₆H₃Cl₂(3,5) | 5 |
| 55 | -C₆H₄-CH₃(p) | -C₆H₂Cl₃(2,3,4) | 5 |
| 56 | -C₆H₄-CH₃(p) | -C₆H₂Cl₃(2,4,6) | 5 |
| 57 | -C₆H₄-CH₃(p) | -C₆H₄-CN(o) | 5 |
| 58 | -C₆H₄-CH₃(p) | -C₆H₄-CH(CH₃)₂(p) | 5 |
| 59 | -C₆H₄-CH₃(p) | -C₆H₄-C(CH₃)₃(p) | 5 |
| 60 | -C₆H₄-CH₃(p) | -C₆H(CH₃)₄(2,3,5,6) | 5 |
| 61 | -C₆H₄-CH₃(p) | -C₆H₄-O-C₈H₁₇ | 5 |
| 62 | -C₆H₄-CH₃(p) | -C₆H₄-O(CH₂)₂-C₆H₅ | 5 |
| 63 | -C₆H₄-CH₃(p) | -C₆H₄-O(CH₂)₃-C₆H₅ | 5 |
| 64 | -C₆H₄-CH₃(p) | -C₆H₄-O(CH₂)₂O-C₆H₅ | 5 |
| 65 | -C₆H₄-CH₃(p) | -C₆H₄-O(CH₂)₃O-C₆H₅ | 5 |
| 66 | -C₆H₄-CH₃(p) | -C₆(CH₃)₅ | 5 |
| 67 | -C₆H₄-CH₃(p) | β-C₁₀H₇ | 5 |
| 68 | -C₆H₄-CH₃(p) | 4-Cl-α-C₁₀H₆ | 5 |
| 69 | -C₆H₄-CH₃(p) | Anthrachinon-2-yl | 5 |
| 70 | -C₆H₄-CH₃(p) | Thiophen-2-yl | 5 |
| 71 | -C₆H₄-CH₃(p) | 2,5-Cl₂-thiophen-3-yl | 5 |
| 72 | -C₆H₄-CH₃(p) | 4,5-Br₂-thiophen-2-yl | 5 |
| 73 | -C₆H₄-CH(CH₃)₂(p) | -C₆H₄F(m) | 5 |
| 74 | -C₆H₄-Buⁿ(p) | -C₆H₂Cl₃(2,3,4) | 5 |
| 75 | -C₆H₄-C(CH₃)₃(p) | -CF₃ | * |
| 76 | -C₆H₃(CH₃)₂(2,5) | -C₆H₄-C(CH₃)₂-C₂H₅(p) | 5 |
| 77 | -C₆H₄F(p) | -CH₃ | 4 |
| 78 | -C₆H₄F(p) | -C₆H₄-CH₃(p) | 5 |
| 79 | -C₆H₄F(p) | -C₆H₄-O(CH₂)₂-C₆H₅ | 5 |
| 80 | -C₆H₄F(p) | -C₆H₄-O(CH₂)₃-C₆H₅ | 5 |
| 81 | -C₆H₄F(p) | -C₆H₄-O(CH₂)₃O-C₆H₅ | 5 |
| 82 | -C₆H₄Cl(p) | -C₆H₄-CH₂-C₂H₅(p) | 5 |
| 83 | -C₆H₄Cl(p) | -C₆HF₂(3,6)Br₂(2,5) | 5 |
| 84 | -C₆H₄Cl(p) | -C₆H₄I(p) | 5 |
| 85 | -C₆H₄Cl(p) | -C₆H₄-O(CH₂)₃-C₆H₅(p) | 5 |
| 86 | -C₆H₄Cl(m) | -C₆H₄-CH₃(p) | 5 |
| 87 | -C₆H₄Cl(o) | -C₆H₄Br(p) | 5 |
| 88 | -C₆H₄Br(p) | -C₆H₄-CN(p) | 5 |
| 89 | -C₆H₄-OCH₃(o) | -C₆H₄-O(CH₂)₂-C₆H₅(p) | 5 |
| 90 | -C₆H₄-OCH₃(o) | -C₆H₃Cl₂(2,3) | 5 |
| 91 | -C₆H₄-OCH₃(o) | α-C₁₀H₇ | 5 |
| 92 | -C₆H₄-OCH₃(p) | -C₂H₅ | 4 |
| 93 | -C₆H₄-OCH₃(p) | -C₆H₄-CH₃(p) | 5 |
| 94 | -C₆H₄-OCH₃(p) | 4,5-Br₂-thiophen-2-yl | 5 |
| 95 | -C₆H₃(OCH₃)₂(3,4) | -C₆H₃Cl₂(3,4) | 5 |
| 96 | -C₆H₄-OBuⁿ | β-C₁₀H₇ | 5 |
| 97 | -C₆H₄-N(CH₃)₂(p) | -C₆H₃F-CH₃(3,5) | 5 |
| 98 | -C₆H₄-CH=CH₂(p) | -CH₃ | 4 |
| 99 | -C₆H₄-CH=CH₂(p) | -C₆H₄-CH₃(p) | 5 |
| 100 | -C₆H₄-CH=CH₂(p) | -C₆H₄-O(CH₂)₂-C₆H₅(p) | 5 |
| 101 | -C₆H₄-CH=CH₂(p) | -C₆H₄-O(CH₂)₃O-C₆H₅(p) | 5 |
| 102 | -C₆H₄-CH=CH₂(p) | -C₆H₄Cl(p) | 5 |
| 103 | -C₆H₄-CH=CH₂(p) | β-C₁₀H₇ | 5 |
| 104 | -C₆H₄-CH=CH₂(m) | -C₂H₅ | 5 |
| 105 | -C₆H₄-CH=CH₂(m) | -C₆H₄-CH₃(p) | 5 |
| 106 | -C₆H₄-CH=CH₂(m) | -C₆H₄-OBuⁿ(p) | 5 |
| 107 | -C₆H₄-CH=CH₂(m) | -C₆H₄-OC₂H₄-C₆H₅(p) | 5 |
| 108 | -C₆H₄-CH=CH₂(m) | -C₆H₄-O(CH₂)₃O-C₆H₅(p) | 5 |
| 109 | -C₆H₄-CH=CH-CH₃(m) | -C₆H₄-CH₃(p) | 5 |
| 110 | -C₆H₄-C≡C-CH₃(p) | -C₆H₅ | 5 |
| 111 | -C₆H₄-C₆H₅(p) | -CH₃ | 4 |
| 112 | -C₆H₄-C₆H₅(p) | -C₂H₅ | 5 |
| 113 | -C₆H₄-C₆H₅(p) | -C₆H₄-Cl(p) | 5 |
| 114 | -C₆H₄-OCH₂-C₆H₅(p) | -C₆H₄Br(p) | 5 |
| 115 | -C₆H₄-OCH₂-C₆H₅(m) | -C₆H₅ | 5 |
| 116 | -C₆H₄-SCH₃(p) | -C₆H₃Cl₂(3,4) | 5 |
| 117 | -C₆H₄-SO₂-CH₃ (p) | -C₂H₅ | 4 |
| 118^{a} | -C₆H₄-O-SO₂-CH₃(p) | -CH₃ | 4 |
| 119^{a} | -C₆H₄-O-SO₂-C₂H₅(p) | -C₂H₅ | 4 |
| 12^{a} | -C₆H₄-NH-SO₂-CH₃(p) | -CH₃ | 4 |
| 121 | -C₆H₄-C(CF₃)₂-OH(p) | -CH₃ | 5 |
| 122 | -C₆H₄-C(CF₃)₂-OH(m) | -CH₂-C₂H₅ | 5 |
| 123 | -C₆H₄-C(CF₃)₂-OH(m) | -C₆H₄Cl(p) | 5 |
| 124 | -C₆H₄-C(CF₃)₂-OH(m) | -C₆H₃Cl₂(3,4) | 5 |
| 125 | -C₆H₄-C(CF₃)₂OH(m) | -C₆H₃-Cl₂(2,5) | 5 |
| 126 | -C₆H₄-C(CF₃)₂OH(m) | -C₆H₄-OCH₃(p) | 5 |

| Nr. | R¹ | R³ | |
|---|---|---|---|
| 127 | -C₆H₄-C(CF₃)₂-OH(m) | -C₆H₃Br(3)-CH₃(4) | |
| 128 | -C₆H₄-C(CF₃)₂-OH(m) | -C₆H₄-NO₂(p) | |
| 128A | -C₆H₃(-C(CF₃)₂-OCH₃)₂(3,5) | -CH₃ | |
| 128B | -C₆H₃(-C(CF₃)₂-OCH₃)₂(3,5) | -C₆H₄-CH₃(p) | |
| 128C | -C₆H₃(-C(CF₃)₂-OCH₃)₂(3,5) | -C₆H₄Cl(p) | |
| 128D | -C₆H₃(-C(CF₃)₂-OCH₃)₂(3,5) | -C₆H₄-NO₂(o) | |
| 128E | -C₆H₃(-C(CF₃)₂-OCH₃)₂(3,5) | β-C₁₀H₇ | |
| 129^{b} | -C₆H₃(-O-SO₂-CH₃)₂(2,4) | -C₆H₅ | |
| 129A | -C₆H₂(-O-SO₂-CH₃)₃(2,3,4) | -CH₃ | |
| 129B | -C₆H₂(-O-SO₂-C₆H₄Cl(p))₃(2,3,4) | -C₆H₄Cl(p) | |
| 130 | β-C₁₀H₇ | -C₆H₄-CH₃(p) | |
| 131 | Thiophen-2-yl | -C₆H₄-CH₃(p) | |
| 132 | Thiophen-2-yl | -C₆H₄-NO₂(m) | |
| 133 | Benzo[b]thiophen-2-yl | -C₆H₄-CH₃(p) | |
| 134 | Benzothiazol-5-yl | -C₆H₄-CH₃(p) | |
| 135 | Chinolin-3-yl | -C₆H₄-CH₃(p) | |

| | | | |
|---|---|---|---|
| Die Verbindungen Nr. 127, 128, 129 und 130 bis 135 sind gemäß Beispiel 5 hergestellt ^{a} Verwendung der doppelten molaren Menge an Sulfonsäurechlorid, da neben der 2-Hydroxypropangruppe gleichzeitig eine OH- bzw. NH-Gruppe umgesetzt wurde. | | | |
| ^{b} Verwendung der dreifachen molaren Menge an Sulfonsäurechlorid, da neben der 2-Hydroxypropangruppe gleichzeitig zwei OH-Gruppen umgesetzt wurden. | | | |
| * Herstellung erfolgte entsprechend G.W. Astrologes et al, J. Amer. Chem. Soc., 99 (1977) 4400. | | | |

Buⁿ = n-Butyl (auch in den folgenden Tabellen)
α-C₁₀H₇ = Naphthalin-1-yl
β-C₁₀H₇ = Naphthalin-2-yl

- Schmelzpunkt der Verbindung:: 128A = 60 °C
128B = 90 °C
128C = 86 °C
128D = 97 °C
128E = 117 °C
129A = 190 °C
129B = 160 °C

**Tabelle 2**

| | | |
|---|---|---|
| Verbindungen der allgemeinen Formel R¹-[CR²(CF₃)-O-SO₂-R³]₂, wobei R² in den Beispielen 136 und 172 -CH₃ und in den übrigen Beispielen -CF₃ ist | | |

| Nr. | R¹ | R³ |
|---|---|---|
| 136 | 1,3-C₆H₄ | -CH₃ |
| 137 | 1,3-C₆H₄ | -C₂H₅ |
| 138 | 1,3-C₆H₄ | -CH₂-C₂H₅ |
| 139 | 1,3-C₆H₄ | -CH(CH₃)₂ |
| 140 | 1,3-C₆H₄ | -[CH₂]₃-CH₃ |
| 141 | 1,3-C₆H₄ | -C₂H₄-O-C₂H₅ |
| 142 | 1,3-C₆H₄ | -CH=CH₂ |
| 143 | 1,4-C₆H₄ | -C₂H₅ |
| 144 | 1,4-C₆H₄ | -CH₂-C₂H₅ |
| 145 | 1,4-C₆H₄ | -CH(CH₃)₂ |
| 146 | 1,4-C₆H₄ | -[CH₂]₃-CH₃ |
| 147 | 1,3-C₆H₄ | -C₆H₅ |
| 148 | 1,3-C₆H₄ | -C₆H₃(CH₃)₂(3,4) |
| 149 | 1,3-C₆H₄ | -C₆H₄Br(p) |
| 150 | 1,3-C₆H₄ | -C₆H₄Cl₂(2,4) |
| 151 | 1,3-C₆H₄ | -C₆H₄Cl₂(3,4) |
| 152 | 1,3-C₆H₄ | -C₆H₄-OBuⁿ(p) |
| 153 | 1,3-C₆H₄ | -C₆H₄-NO₂(m) |
| 154 | 1,3-C₆H₄ | -C₆H₄-NO₂(p) |
| 155 | 1,3-C₆H₄ | -CH₂-C₆H₅ |
| 156 | 1,3-C₆H₄ | -CH=CH-C₆H₅ |
| 157 | 1,3-C₆H₄ | -C₆H₄-O(CH₂)₂-C₆H₅(p) |
| 158 | 1,3-C₆H₄ | -C₆H₄-O(CH₂)₂O-C₆H₅(p) |
| 159 | 1,3-C₆H₄ | -C₆H₄-O(CH₂)₃O-C₆H₅(p) |
| 160 | 1,3-C₆H₄ | β-C₁₀H₇ |
| 161 | 1,3-C₆H₄ | 4-C₆H₅-SO₂-thiophen-2-yl |
| 162 | 1,4-C₆H₄ | -C₆H₅ |
| 163 | 1,4-C₆H₄ | -C₆H₄Cl(p) |
| 164 | 1,4-C₆H₄ | -C₆H₄-CH₃(p) |
| 165 | 1,4-C₆H₄ | -CH₂-C₆H₅ |
| 166 | 4-CH₃-1,3-C₆H₃ | -CH₃ |
| 167 | 4-CH₃-1,3-C₆H₃ | -C₂H₅ |
| 168 | 4-CH₃-1,3-C₆H₃ | -C₆H₄-CH₃(p) |
| 169 | 4-CH₃-1,3-C₆H₃ | -C₆H₄Cl(p) |
| 170 | 4-CH₃-1,3-C₆H₃ | -C₆H₄-O(CH₂)₂O-C₆H₅(p) |
| 171^{a} | 4-CH₃SO₃-1,3-C₆H₄ | -C₆H₄-CH₃(p) |
| 172 | 4,4'-C₆H₄-C₆H₄- | -CH₃ |
| 173 | 4,4'-C₆H₄-C₆H₄- | -CH₃ |
| 174 | 4,4'-C₆H₄-C₆H₄- | -CH₂-C₂H₅ |
| 175 | 4,4'-C₆H₄-C₆H₄- | -C₆H₄Cl(p) |
| 176 | 4,4'-C₆H₄-C₆H₄- | -C₆H₄-OC₂H₅ |
| 177 | 4,4'-C₆H₄-C₆H₄- | -β-C₁₀H₇ |
| 178 | 4,4'-C₆H₄-C₆H₄- | Benzo[b]thiophen-2-yl |
| 179 | 4,4'-C₆H₄-O-C₆H₄- | -CH₃ |
| 180 | 4,4'-C₆H₄-O-C₆H₄- | -C₆H₄-CH₃(p) |
| 181 | 3,3'-C₆H₄-C(CH₃)₂-C₆H₄- | -CH₃ |
| 182 | 3,3'-C₆H₄-C(CH₃)₂-C₆H₄- | -C₆H₄Cl(p) |
| 183 | 3,3'-C₆H₄-SO₂-C₆H₄- | -CH₃ |
| 184 | 3,3'-C₆H₄-SO₂-C₆H₄- | -C₆H₄-CH₃(p) |
| 185 | 3,3'-C₆H₄-CO-C₆H₄- | -CH₃ |
| 186 | 3,3'-C₆H₄-CO-C₆H₄- | -C₆H₄-CH₃(p) |
| 187 | 3,3'-C₆H₄-C(CF₃)₂-C₆H₄- | -CH₃ |
| 188 | 3,3'-C₆H₄-C(CF₃)₂-C₆H₄- | -C₆H₄-CH₃(p) |
| 189 | 3,3'-C₆H₄-C(CF₃)₂-C₆H₄- | -C₆H₄-O(CH₂)₂-OC₆H₅(p) |

| | | |
|---|---|---|
| ^{a} hergestellt unter Verwendung der dreifachen molaren Menge an Sulfonsäurechlorid, da neben den zwei aliphatischen Hydroxygruppen noch eine phenolische Hydroxygruppe zu verestern war. | | |

**Tabelle 3**

| | | | |
|---|---|---|---|
| Verbindungen der allgemeinen Formel R³-[SO₂-O-C(CF₃)(R²)-R¹]ₙ, in denen R² = -CF₃ ist. | | | |

| Nr. | R¹ | n | R³ |
|---|---|---|---|
| 190 | -C₆H₅ | 2 | -CH₂- |
| 191 | -C₆H₄-CH₃(p) | 2 | -CH₂- |
| 192 | -C₆H₄-OCH₃(o) | 2 | -CH₂- |
| 193 | -C₆H₄F(p) | 2 | -C₃H₆- |
| 194 | -C₆H₄F(p) | 2 | -C₄H₈- |
| 195 | -C₆H₅ | 2 | 1,3-C₆H₄- |
| 196 | -C₆H₄-CH₃(p) | 2 | 1,3-C₆H₄- |
| 197 | -C₆H₄-CH(CH₃)₂(p) | 2 | 1,3-C₆H₄- |
| 198 | -C₆H₃(CH₃)₂(2,4) | 2 | 1,3-C₆H₄- |
| 199 | -C₆H₄F(p) | 2 | 1,3-C₆H₄- |
| 200 | -C₆H₄Cl(p) | 2 | 1,3-C₆H₄- |
| 201 | -C₆H₄-C₆H₅(p) | 2 | 1,3-C₆H₄- |
| 202 | -C₆H₅ | 2 | 2,4,6-(CH₃)₃-1,3-C₆H- |
| 203 | -C₆H₄Cl(m) | 2 | 2,4,6-(CH₃)₃-1,3-C₆H- |
| 204 | -C₆H₅ | 2 | 1,5-C₁₀H₆-* |
| 205 | -C₆H₄-CH₃(p) | 2 | 1,5-C₁₀H₆-* |
| 206 | -C₆H₅ | 2 | 2,6-C₁₀H₆- |
| 207 | -C₆H₄-CH₃(p) | 2 | 2,6-C₁₀H₆- |
| 207A | -CH₃ | 3 | 1,3,5-C₆H₃*** |
| 207B | -CH₂-C₂H₅ | 3 | 1,3,5-C₆H₃*** |
| 207C | -C₆H₄Cl(p) | 3 | 1,3,5-C₆H₃*** |
| 207D | β-C₁₀H₇ | 3 | 1,3,5-C₆H₃*** |
| 208 | -C₆H₅ | 3 | 1,3,7-C₁₀H₅-** |
| 209 | -C₆H₄-CH₃(p) | 3 | 1,3,7-C₁₀H₅-** |

| | | | |
|---|---|---|---|
| * 1,5-C₁₀H₆ = Naphthalin-1,5-diyl | | | |
| ** 1,3,7-C₁₀H₅ = Naphthalin-1,3,7-triyl | | | |
| *** Benzol-1,3,5-triyl | | | |

Die nachstehend beschriebenen Beispiele stellen lediglich eine Auswahl des Erfindungsgedankens dar. Dieser soll daher nicht auf die Beispiele begrenzt sein.

### Beispiel 1:

Eine Beschichtungslösung wurde hergestellt aus

| | |
|---|---|
| 6,86 Gt | eines Poly(4-hydroxystyrols) mit einem mittleren Molekulargewicht (M_{W}) von 18.800 g/mol, |
| 2,94 Gt | eines Poly-N,O-acetals aus Benzaldehyd und n-Propylcarbamidsäure-2-hydroxyethylester (M_{W} = 8.700 g/mol) |
| 0,2 Gt | 4-Chlor-benzolsulfonsäure-(2,2,2-trifluor-1-trifluormethyl-1-(3-vinyl-phenyl)-ethylester) (s. Herstellungsbeispiel 5), und |
| 40 Gt | Propylenglykol-monomethylether-acetat. |

Die Lösung wurde durch einen Filter mit einem Porendurchmesser von 0,2 µm filtriert und auf einen mit einem Haftvermittler (Hexamethyldisilazan) behandelten Siliciumwafer so aufgeschleudert, daß nach der Trocknung bei 120 °C für 1 min auf der hot plate wurde eine Schichtdicke von 1,04 µm erhalten wurde.

Das positiv arbeitende Aufzeichnungsmaterial wurde bildmäßig unter einer Vorlage mit der UV-Strahlung einer Xenon-Quecksilberdampflampe bei 254 nm mit einer Energie von 8 mJ/cm² belichtet. 4 min nach der Belichtung wurde das Gemisch auf einer hot plate 60 s lang auf 60 °C nacherwärmt.

Anschließend wurde das Aufzeichnungsmaterial mit einer 0,21 N wäßrigen Lösung von Tetramethylammoniumhydroxid (TMAH) entwickelt. Nach einer Entwicklungsdauer von 60 s bei 21 °C waren die bestrahlten Bereiche restlos abgelöst. Die Maskenvorlage war genau abgebildet worden. Selbst die kleinen Strukturen der Maske von 350 nm Größe und darunter waren noch detailgetreu abgebildet.

### Beispiel 2:

Eine Beschichtungslösung wurde hergestellt aus

| | |
|---|---|
| 7,0 Gt | eines Copolymeren aus 2 Teilen 3-Methyl-4-hydroxystyrol und 1 Teil 4-Hydroxystyrol (M_{W} = 14.000 g/mol) mit einer OH-Zahl von 425 mg/g, |
| 3,0 Gt | eines Poly-N,O-acetals aus 1 Teil Benzaldehyd und 1 Teil Propylcarbamidsäure-2-hydroxyethylester (M_{W} = 5.000), |
| 0,25 Gt | 4-Chlor-benzolsulfonsäure-(2,2,2-trifluor-1-p-tolyl-1-trifluormethyl-ethylester) (Verbindung Nr. 49 in Tabelle 1), und |
| 40 Gt | Propylenglykol-monomethylether-acetat. |

Die Lösung wurde wie in Beispiel 1 beschrieben vorbehandelt und auf den Wafer aufgetragen. Nach dem Trocknen verblieb eine Schicht mit einer Dicke von 1,02 µm.

Das Aufzeichnungsmaterial wurde bildmäßig unter einer Vorlage mit der UV-Strahlung einer Xenon-Quecksilberdampflampe bei 254 nm mit einer Energie von 20 mJ/cm² belichtet. Nach 4 min wurde das Gemisch auf einer hot plate 60 s auf 60 °C nacherwärmt.

Anschließend wurde das Aufzeichnungsmaterial mit einer 0,27 N wäßrigen TMAH-Lösung entwickelt. Wieder war die Maskenvorlage bis herab zu Strukturen von 300 nm Größe genau abgebildet.

### Beispiel 3:

Ein wie in Beispiel 1 beschichteter Wafer wurde unter einer Vorlage mit der Uv-Strahlung eines KrF-Excimer-Lasers (248 nm) mit einer Energie von 9 mJ/cm² bestrahlt. Das Material wurde 4 min gelagert und anschließend wie oben beschrieben nacherwärmt und entwickelt. Das Auflösungsvermögen des Aufzeichnungsmaterial erwies sich als exzellent. Die Kantensteilheit bei 350 nm Strukturen war besser als 87°.

### Beispiel 4:

Ein wie in Beispiel 1 beschichteter Wafer wurde unter einer Vorlage mit der Uv-Strahlung eines KrF-Excimer-Lasers (248 nm) mit einer Energie von 9 mJ/cm² (gleiche Dosis wie in Beispiel 3) bestrahlt und vor der Nacherwärmung 60 min bei Raumtemperatur gelagert. Bei der Entwicklung erhielt man ein Abbild, das dem des Beispiels 3 entsprach. Ein Einfluß der Lagerzeit zwischen Belichtung und Nacherwärmung auf die Belichtungsdosis ließ sich somit nicht feststellen.

### Beispiel 5:

Eine Beschichtungslösung wurde hergestellt aus

| | |
|---|---|
| 7,5 Gt | eines Copolymeren aus Styrol und 4-Hydroxystyrol (20/80) (M_{W} = 28.000), |
| 3,1 Gt | Piperonal-bis(phenoxyethyl)-acetal, |
| 0,45 Gt | 4-(2-Phenoxy-ethoxy)-benzolsulfonsäure-(2,2,2-trifluor-1-p-tolyl-1-trifluormethyl-ethylester) (Verbindung Nr. 64), und |
| 30 Gt | Propylenglykol-monomethylether-acetat. |

Die Lösung wurde wie in Beispiel 2 beschrieben vorbehandelt und beschichtet. Der 1,2 µm dicke Film wurde bildmäßig unter einer Vorlage mit der Uv-Strahlung einer Xenon-Quecksilberdampflampe bei 254 nm mit einer Energie von 28 mJ/cm² belichtet. Nach 5 min wurde das Gemisch auf einer hot plate 60 s auf 70 °C nacherwärmt.

Anschließend wurde das Aufzeichnungsmaterial mit einer 0,18 N wäßrigen TMAH-Lösung entwickelt. Strukturen von weniger als 500 nm Größe waren detailgetreu abgebildet. Der Kontrast des Materials war > 4.

### Beispiel 6:

Eine Beschichtungslösung wurde hergestellt aus

| | |
|---|---|
| 7,0 Gt | eines Homopolymeren aus 3-Methyl-4-hydroxystyrol mit einem mittleren Molekulargewicht von 17.000 und einer OH-Zahl von 385 mg/g, |
| 3,0 Gt | eines Poly-N,O-acetals aus 1 Teil Benzaldehyd und 1 Teil Propylcarbamidsäure-2-hydroxyethylester (M_{W} = 3.500), |
| 0,2 Gt | Naphthalin-2-sulfonsäure-[2,2,2-trifluor-1-trifluormethyl-1-(4-vinyl-phenyl)-ethylester] (Verbindung Nr. 103 in Tabelle 1), und |
| 40 Gt | Propylenglykol-monomethylether-acetat. |

Nach Standard-Vorbehandlung wurde der resultierende 1,02 µm dicke Film unter einer Vorlage KrF-Excimer-Laser Strahlung einer Dosis von 18 mJ/cm² ausgesetzt. Nach 5 min wurde das Gemisch auf einer hot plate 60 s auf 60 °C nacherwärmt.

Anschließend wurde das Aufzeichnungsmaterial mit einer 0,27 N wäßrigen TMAH-Lösung entwickelt. Die Maskenvorlage war wie im voranstehenden Beispiel beschrieben genau abgebildet. Selbst Strukturen < 400 nm waren detailgetreu abgebildet.

### Beispiel 7:

Die Beschichtungslösung des Beispiels 6 wurde dahingehend verändert, daß anstelle des Poly-N,O-Acetals ein Polyorthoester, hergestellt durch Kondensation von Orthoameisensäure-trimethylester und 7,7-Bis-hydroxymethyl-nonan-1-ol, verwendet wurde.

Das Gemisch wurde nach der üblichen Vorbehandlung bei 110 °C zu einer Filmdicke von 1,06 µm getrocknet.

Das Aufzeichnungsmaterial wurde bildmäßig unter einer Vorlage mit der UV-Strahlung einer Xenon-Quecksilberdampflampe bei 254 nm mit einer Energie von 11 mJ/cm² belichtet. Der Wafer wurde nach 5 min auf einer hot plate 60 s auf 65 °C nacherwärmt.

Anschließend wurde mit einer 0,24 N wäßrigen TMAH-Lösung entwickelt. Das Material löste die Vorlagen der Maske bis herab zu Strukturen von 600 nm auf.

### Beispiel 8:

Eine Beschichtungslösung wurde hergestellt aus

| | |
|---|---|
| 6,5 Gt | eines Copolymeren aus 1,5 Teilen 3-Methyl-4-hydroxystyrol und 1 Teil 4-Hydroxystyrol mit einem mittleren Molekulargewicht von 14.000 und einer OH-Zahl von 410 mg/g, |
| 3,5 Gt | eines Poly-N,O-acetals aus 1 Teil Benzaldehyd und 1 Teil Propylcarbamidsäure-2-hydroxyethylester |
| 0,3 Gt | Propansulfonsäure-(2,2,2-trifluor-1-phenyl-1-trifluormethyl-ethylester) (Verbindung Nr. 32 in Tabelle 1A), und |
| 40 Gt | Propylenglykol-monomethylether-acetat. |

Die Lösung wurde durch einen Filter mit einem Porendurchmesser von 0,2 µm filtriert und auf zwei mit einem Haftvermittler (Hexamethyldisilazan) behandelten Siliciumwafer bei 3.000 Umdrehungen aufgeschleudert. Nach der Trocknung auf 120 °C für 1 min auf der hot plate wurde eine Schichtdicke von 0,99 +/- 0,03 µm erhalten.

Das Aufzeichnungsmaterial wurde bildmäßig unter einer Vorlage mit der UV-Strahlung einer Xenon-Quecksilberdampflampe bei 254 nm mit einer Energie von 16 mJ/cm² belichtet. Der eine Wafer wurde nach 5 min, der zweite Wafer nach 60 min auf einer hot plate 60 s auf 60 °C nacherwärmt.

Anschließend wurden die belichteten Wafer mit einer 0,24 N wäßrigen TMAH-Lösung entwickelt. Die Maskenvorlage war ähnlich wie in den voranstehenden Beispiel beschrieben genau abgebildet. In beiden Fällen wurden 350 nm Strukturen klar aufgelöst. Die Veränderung der Linienbreite war nur geringfügig.

### Beispiel 9:

Eine Beschichtungslösung wurde hergestellt aus

| | |
|---|---|
| 6,5 Gt | eines Copolymeren aus 2 Teilen 3-Methyl-4-hydroxystyrol und 1 Teil 4-Hydroxystyrol mit einem mittleren Molekulargewicht von 14.000, |
| 3,5 Gt | eines Poly-N,O-acetals aus 1 Teil Benzaldehyd und 1 Teil Propylcarbamidsäure-2-hydroxyethylester |
| 0,4 Gt | 4-Butoxy-benzolsulfonsäure-[2,2,2-trifluor-1-trifluormethyl-1-(3-vinyl-phenyl)-ethylester] (Verbindung Nr. 106 in Tabelle 1A), und |
| 40 Gt | Propylenglykol-monomethylether-acetat. |

Die Lösung wurde durch einen Filter mit einem Porendurchmesser von 0,2 µm filtriert und auf drei mit einem Haftvermittler (Hexamethyldisilazan) behandelte Siliciumwafer bei 3.000 Umdrehungen aufgeschleudert. Nach der Trocknung auf 120 °C für 1 min auf der hot plate wurde eine Schichtdicke von 1,02 +/- 0,03 µm erhalten.

Das Aufzeichnungsmaterial wurde bildmäßig unter einer Vorlage mit der UV-Strahlung eines KrF-Excimer Lasers mit einem Energiefächer von 15 bis 22 mJ/cm² belichtet. Der erste Wafer wurde nach 5 min, der zweite Wafer nach 60 min und der dritte nach 120 min auf einer hot plate 60 s auf 60 °C nacherwärmt.

Anschließend wurden die belichteten Wafer mit einer 0,27 N wäßrigen TMAH-Lösung entwickelt. In allen drei Fällen wurden die besten Ergebnisse bei einer konstanten Belichtungsdosis von 18 mJ/cm² erhalten: die Flanken waren senkrecht und die Auflösungsgrenze betrug 0,25 µm. Beim 120 min gelagerten Wafer war eine geringfügig stärkere Unterschneidung der Strukturen zu erkennen.

### Beispiel 10:

Der Beschichtungslösung des Beispiels 9 wurden 0,3 Gt eines Amins (^{(R)}Tinuvin 440) zugefügt. Im weiteren Verlauf wurde wie in Beispiel 9 beschrieben vorgegangen, wobei zur maßgetreuen Wiedergabe der Maske eine Belichtungsdosis von 24 mJ/cm² erforderlich war. Wieder ließen sich alle Wafer bei dieser Dosis in gleicher Qualität entwickeln. Eine Unterschneidung wurde auch nach 120 min nicht beobachtet.

### Beispiel 11:

Das Aufzeichnungsmaterial des Beispiels 9 wurde bildmäßig Elektronenstrahlung einer Energie von 8,32 µC/cm² (20 keV) ausgesetzt. Das Schreibmuster war nach der in Beispiel 9 beschriebenen Verarbeitung klar zu erkennen.

### Beispiel 12:

Eine Beschichtungslösung wurde hergestellt aus

| | |
|---|---|
| 9,6 Gt | eines Copolymers aus Brenzkatechin-monomethacrylat und tert.-Butoxycarbonyl-brenzkatechinmonomethacrylat (25 : 75), |
| 0,5 Gt | 3,5-Dichlor-benzolsulfonsäure-(2,2,2-trifluor-1-p-tolyl-1-trifluormethyl-ethylester) (Verbindung Nr. 54) in |
| 40 Gt | Propylenglykol-monomethylether-acetat. |

Nach einer entsprechend Beispiel 1 durchgeführten Vorbehandlung wurde durch Trocknen bei 110 °C für 1 min ein Film mit einer Dicke von 0,8 µm erhalten.

Das Aufzeichnungsmaterial wurde entsprechend Beispiel 2 mit einer Dosis von 11 mJ/cm² belichtet, für 1 min auf 100 °C nacherwärmt und für 1 min in einen 0,3 N wäßrigen TMAH Entwickler getaucht. Man erhielt eine exakte Reproduktion der Vorlage, wobei auch Strukturen kleiner als 0,5 µm einwandfrei aufgelöst waren.

### Beispiel 13:

Eine Beschichtungslösung wurde hergestellt aus

| | |
|---|---|
| 6,86 Gt | eines Poly(4-hydroxystyrols) mit einem mittleren Molekulargewicht (M_{W}) von 18.800 g/mol, |
| 2,94 Gt | eines Poly-N,O-acetals aus Benzaldehyd und n-Propylcarbamidsäure-2-hydroxyethylester (M_{W} = 8.700 g/mol) |
| 0,2 Gt | 1,3-Bis-(2,2,2-trifluor-1-methansulfonyloxy-1-trifluormethyl-ethyl) -benzol (Verbindung des Herstellungsbeispiels Nr. 6) und |
| 40 Gt | Propylenglykolmonomethyletheracetat. |

Die Lösung wurde durch einen Filter mit einem Porendurchmesser von 0,2 µm filtriert und auf einen mit einem Haftvermittler (Hexamethyldisilazan) behandelten Siliziumwafer so aufgeschleudert, daß nach der Trocknung bei 120 °C für 1 min auf der hot plate eine Schichtdicke von 1,06 µm erhalten wurde.

Das positiv arbeitende Aufzeichnungsmaterial wurde bildmäßig unter einer Vorlage mit der UV-Strahlung einer Xenon-Quecksilberdampflampe bei 254 nm mit einer Energie von 8,6 mJ/cm² belichtet. 4 min nach der Belichtung wurde das Gemisch auf einer hot plate 60 s auf 60 °C nacherwärmt.

Anschließend wurde das Aufzeichnungsmaterial mit einer 0,21 N wäßrigen TMAH-Lösung entwickelt. Nach 60 s bei 21°C waren dabei die bestrahlten Bereiche restlos abgelöst. Die Maskenvorlage war in dem erhaltenen Positivbild genau abgebildet worden. Selbst die kleinen Strukturen der Maske von 350 nm Größe und darunter waren noch detailgetreu abgebildet.

### Beispiel 14:

Eine Beschichtungslösung wurde hergestellt aus

| | |
|---|---|
| 8,0 Gt | eines Copolymeren aus 3-Methyl-4-hydroxystyrol/4-Hydroxystyrol (Molverhältnis 75 : 25) mit einem mittleren Erweichungspunkt von > 150°C und einem mittleren Molekulargewicht von 28.000 |
| 2,0 Gt | Hexa-N-methoxymethylmelamin und |
| 0,5 Gt | 1,4-Bis-(2,2,2-trifluor-1-methansulfonyloxy-1-trifluormethyl-ethyl)-benzol (Verbindung des Herstellungsbeispiels Nr. 8) in |
| 42 Gt | Propylenglykol-monomethylether-acetat. |

Das Gemisch wurde wie in Beispiel 1 beschrieben vorbehandelt, auf einen Wafer aufgebracht und bei 110 °C für 1 min zu einer Filmdicke von 1,01 µm getrocknet.

Die Belichtung erfolgte durch eine Negativvorlage mit Strahlung einer Xenon-Quecksilberdampflampe (254 nm) mit einer Dosis von 4,2 mJ/cm². Anschließend wurde das Gemisch auf der hot plate 2 min auf 120 °C erwärmt.

Das Gemisch wurde 1 min in einen 0,27 N wäßrigen TMAH Entwickler getaucht, wobei auch feinste unbelichtete Details der Vorlage abgelöst wurden. Das erhaltene negative Abbild zeigte ein Auflösungsvermögen bis herab zu Strukturen von 0,35 µm.

### Beispiel 15

Es wurde eine Beschichtungslösungen hergestellt aus

| | |
|---|---|
| 7,0 Gt | des in Beispiel 1 beschriebenen Copolymeren |
| 3,0 Gt | des in Beispiel 1 beschriebenen Poly-N,O-acetals |
| 0,4 Gt | 1,3-Bis-(2,2,2-trifluor-1-methansulfonyloxy-1trifluormethy-ethyl)-benzol gemäß Herstellungsbeispiel 6 als Methansulfonsäure erzeugender Photosäuregenerator und |
| 40 Gt | Propylenglykol-monomethylether-acetat. |

### Vergleichsbeispiele 16 bis 21:

Es wurden Beschichtungslösungen gemäß Beispiel 15 hergestellt, in denen der Photosäuregenerator wie folgt ersetzt wurde:
- 16:: 2,6-Dinitrobenzylmethansulfonat
- 17:: Benzoinmethansulfonat
- 18:: Pyrogalloltrismethansulfonat
- 19:: N-Methansulfonyloxy-succinimid
- 20:: 1-Methansulfonyloxy-4-methyl-6-phenyl-pyridin-2-on
- 21:: 2-Methansulfonyloxy-2-p-tolyl-propan

Die Lösungen gemäß Beispiel 15 und Vergleichsbeispielen 16 bis 21 wurden filtriert und auf je zwei Wafer aufgeschleudert. Nach Trocknung bei 110 °C lag die Schichtdicke in allen Fällen bei 1,0 +/- 0,04 µm.

Die Durchlässigkeit der Filme für Strahlung einer Wellenlänge von 248 nm nahm in der Reihenfolge 17 < 16 < 20 < 21 < 15 = 18 = 19 zu, d.h. nur die Filme der Vergleichsbeispiele 18 und 19 waren ähnlich vorteilhaft transparent wie bei Beispiel 15.

Die Aufzeichnungsmaterialien wurden bildmäßig unter einer Vorlage mit der UV-Strahlung einer Xenon-Quecksilberdampflampe bei 254 nm belichtet. Die zur Abbildung notwendige Belichtungsdosis betrug zwischen 5,5 mJ/cm² und 75 mJ/cm² (5,5 mJ/cm² = 15 = 19 < 18 < 20 < 16 < 17 << 21 = 75 mJ/cm²). Je einer der Wafer wurde nach 5 min, der zweite Wafer nach 60 min auf einer hot plate 60 s auf 60 °C nacherwärmt.

Anschließend wurden die belichteten Wafer mit einer 0,27 N wäßrigen TMAH-Lösung entwickelt. Bei den 5 min gelagerten Materialien war die Maskenvorlage bis herab zu 400 nm von allen Materialien wiedergegeben. Strukturen < 400 nm wurden von den Aufzeichnungsmaterialien der Beispiele 15, 18, 19, und 21 aufgelöst. Bei den mit der gleichen Dosis belichteten 60 min gelagerten Wafern konnten Abbildungen im Bereich von 450 nm nur mit den Aufzeichnungsmaterialien der Beispiele 15 und 18 erhalten werden, bei allen anderen waren die Strukturen dieser Größe vollständig weggelöst. Strukturen im Bereich von 350 nm und kleiner wurden nur vom Aufzeichnungsmaterial des Beispiels 15 mit allerdings deutlich erkennbarem Unterschnitt wiedergegeben. Aus diesen Ergebnissen ergibt sich, daß das latente Bild der Aufzeichnungsmaterialien bei Verwendung des erfindungsgemäßen Gemisches erheblich stabilisiert ist und nur mit diesen eine von der Lagerzeit unabhängige Konstanz der Belichtungsdosis gewährleistet ist. Das mit den erfindungsgemäßen Gemischen erhaltene Ergebnis läßt sich noch weiter verbessern, wenn basische Zusätze, z.B. Amine, zum Gemisch hinzugefügt werden (vgl. Beispiel 10).

### Beispiel 22:

Eine Beschichtungslösung wurde hergestellt aus

| | |
|---|---|
| 8,0 Gt | eines Copolymeren aus 3-Methyl-4-hydroxystyrol/4-Hydroxystyrol (Molverhältnis 75 : 25) mit einem mittleren Erweichungspunkt von > 150 °C und einem mittleren Molekulargewicht von 28.000 |
| 2,0 Gt | Hexa-N-methoxymethyl-melamin und |
| 0,5 Gt | 5-Chlor-thiophen-2-sulfonsäure-(2,2,2-trifluor-1-phenyl-1-trifluormethyl-ethylester) (Verbindung Nr. 42) in |
| 42 Gt | Propylenglykol-monomethylether-acetat. |

Das Gemisch wurde wie in Beispiel 1 beschrieben vorbehandelt, auf einen Wafer aufgebracht und bei 110 °C für 1 min zu einer Filmdicke von 1,03 µm getrocknet. Die Belichtung erfolgte durch eine Negativvorlage mit Strahlung einer Xenon-Quecksilberdampflampe (254 nm) mit einer Dosis von 3,8 mJ/cm². Anschließend wurde das Gemisch auf der hot plate 2 min auf 120 °C erwärmt.

Das Gemisch wurde 1 min in einen 0,27 N wäßrigen TMAH Entwickler getaucht, wobei auch feinste unbelichtete Details der Vorlage abgelöst wurden. Das erhaltene Negativabbild zeigte ein Auflösungsvermögen bis herab zu Strukturen von 0,35 µm.

### Beispiel 23:

Eine Beschichtungslösung wurde hergestellt aus

| | |
|---|---|
| 7,5 Gt | eines Homopolymers aus 3-Methyl-4-hydroxystyrol mit einem mittleren Molekulargewicht von 14.000 |
| 2,5 Gt | 4,4'-Bis-methoxymethyl-diphenylether und |
| 0,5 Gt | 4-(3-Phenyl-propoxy)-benzolsulfonsäure-[1-(4-chlor-phenyl)-2,2,2-trifluor-1-trifluormethylethylester] (Verbindung Nr. 85) in |
| 42 Gt | Propylenglykolmonomethylether. |

Das Gemisch wurde wie in Beispiel 1 beschrieben vorbehandelt, auf einen Wafer aufgebracht und bei 110 °C für 1 min zu einer Filmdicke von 0,98 µm getrocknet.

Die Belichtung erfolgte durch eine Negativvorlage mit Strahlung einer Xenon-Quecksilberdampflampe (254 nm) mit einer Dosis von 7,6 mJ/cm². Anschliepend wurde das Gemisch auf der hot plate 2 min auf 115 °C erwärmt.

Das Gemisch wurde 1 min in einen 0,27 N wäßrigen TMAH Entwickler getaucht, wobei auch feinste unbelichtete Details der Vorlage abgelöst wurden. Das erhaltene Negativabbild zeigte ein Auflösungsvermögen bis herab zu Strukturen von 0,45 µm.

### Beispiele 24 bis 72:

Es wurden Beschichtungslösungen analog zu Beispiel 1 (Tonalität +) oder Beispiel 22 (Tonalität -) hergestellt, wobei die in der nachstehenden Tabelle genannten Sulfonsäureester in gleicher Gewichtsmenge eingesetzt wurden. Die Verarbeitung erfolgte wie in den Beispielen angegeben. Nachstehend werden Empfindlichkeit und Auflösungsvermögen kleiner Linien (nicht optimiert) angegeben. Die Beispiele sollen belegen, daß praktisch mit allen erfindungsgemäßen Verbindungen der Formeln I und/oder II hochempfindliche Aufzeichnungsmaterialen herstellbar sind, und ihre Empfindlichkeit hauptsächlich durch die Gegenwart der -C(CF₃)R-O-SO₂-Gruppe bestimmt wird.

| Nr. | Verbindung Nr. | Tonalität | Empfindlichkeit (mJ/cm²) | Auflösung (µm) |
|---|---|---|---|---|
| 24 | 18 | + | 32 | < 0,4 |
| 25 | 23 | + | 22 | 0,4 |
| 26 | 28 | + | 25 | < 0,4 |
| 27 | 34 | - | 16 | < 0,4 |
| 28 | 39 | + | 22 | < 0,4 |
| 29 | 40 | + | 16 | < 0,4 |
| 30 | 45 | + | 28 | < 0,4 |
| 31 | 46 | + | 35 | 0,4 |
| 32 | 49 | + | 17 | < 0,4 |
| 33 | 52 | + | 14 | < 0,4 |
| 34 | 55 | + | 12 | < 0,4 |
| 35 | 61 | + | 45 | 0,4 |
| 36 | 64 | + | 38 | < 0,4 |
| 37 | 68 | + | 32 | < 0,4 |
| 38 | 79 | + | 29 | < 0,4 |
| 39 | 83 | - | 14 | < 0,4 |
| 40 | 94 | - | 21 | 0,4 |
| 41 | 98 | + | 8,2 | < 0,4 |
| 42 | 101 | + | 11 | < 0,4 |
| 43 | 103 | + | 12 | < 0,4 |
| 44 | 104 | + | 7,5 | < 0,4 |
| 45 | 108 | + | 10 | < 0,4 |
| 46 | 111 | + | 7 | < 0,4 |
| 47 | 114 | + | 9,5 | < 0,4 |
| 48 | 125 | + | 8,2 | < 0,4 |
| 49 | 130 | + | 18 | 0,4 |
| 50 | 134 | + | 22 | 0,4 |
| 51 | 138 | + | 11 | < 0,4 |
| 52 | 142 | + | 10 | < 0,4 |
| 53 | 149 | + | 6 | < 0,4 |
| 54 | 151 | + | < 6 | < 0,4 |
| 55 | 156 | + | 8 | < 0,4 |
| 56 | 158 | + | 11 | < 0,4 |
| 57 | 158 | - | 9 | < 0,4 |
| 58 | 160 | + | 12 | < 0,4 |
| 59 | 166 | + | 8 | < 0,4 |
| 60 | 169 | + | 4,5 | < 0,4 |
| 61 | 173 | + | 11 | < 0,4 |
| 62 | 179 | + | 9 | < 0,4 |
| 63 | 183 | + | 7 | < 0,4 |
| 64 | 183 | - | 6 | < 0,4 |
| 65 | 187 | + | 7 | < 0,4 |
| 66 | 187 | - | 6 | < 0,4 |
| 67 | 189 | + | 12 | < 0,4 |
| 68 | 193 | + | 22 | 0,4 |
| 69 | 196 | + | 19 | < 0,4 |
| 70 | 196 | - | 13 | < 0,4 |
| 71 | 202 | + | 19 | < 0,4 |
| 72 | 207 | + | 17 | 0,4 |

## Patentansprüche

1. Strahlungsempfindliches, positiv oder negativ arbeitendes Gemisch mit
a) einer bei Bestrahlung eine starke Säure bildenden Verbindung,
b) entweder, für ein positiv arbeitendes Gemisch, einer Verbindung mit mindestens einer durch Säure spaltbaren C-O-C- oder C-O-Si-Bindung, oder, für ein negativ arbeitendes Gemisch, einer Verbindung mit mindestens zwei durch Säure vernetzbaren Gruppen und
c) einem in Wasser unlöslichen, in wäßrig-alkalischen Lösungen dagegen löslichen oder zumindest quellbaren Bindemittel,
dadurch gekennzeichnet, daß die Verbindung a) ein Sulfonsäureester der Formel
[R¹-CR²(CF₃)-O-SO₂-]ₙR³ (I)
oder
R¹[-CR²(CF₃)-O-SO₂-R³]ₘ (II)
ist, worin
R¹ in den Verbindungen der Formel I ein ein- oder mehrkerniger nichtkondensierter oder kondensierter (C₆-C₁₄)Aryl- oder (C₄-C₁₁)Heteroarylrest mit Sauerstoff, Schwefel oder Stickstoff als Heteroatom und
in den Verbindungen der Formel II ein zwei- oder dreiwertiger Rest eines ein- oder mehrkernigen nichtkondensierten oder kondensierten (C₆-C₁₄)Aromaten oder (C₄-C₁₂)Heteroaromaten mit Sauerstoff, Schwefel oder Stickstoff als Heteroatom oder ein zweiwertiger Rest der Formel -C₆H₄-X-C₆H₄-, worin X für ein Sauerstoffatom, eine Carbonyl- oder Sulfonylgruppe oder eine Gruppe CR⁷R⁸, in der R⁷ und R⁸ gleich oder verschieden sind und einen Methyl- oder Trifluormethylrest bedeuten,
R² ein Wasserstoffatom, ein Methyl-, Trifluormethyl- oder Cyanorest,
R³ in den Verbindungen der Formel I mit n = 1 sowie in den Verbindungen der Formel II ein geradkettiger oder verzweigter, unsubstituierter oder substituierter (C₁-C₁₈)Alkylrest, ein (C₄-C₁₀)Cycloalkylrest, ein (C₂-C₆)Alkenylrest, ein unsubstituierter oder substituierter (C₆-C₁₄)Aryl- oder (C₄-C₁₂)Heteroarylrest mit Sauerstoff, Schwefel oder Stickstoff als Heteroatom oder ein (C₇-C₁₈)Aralkylrest,
in den Verbindungen der Formel I mit n = 2 oder 3 ein zwei- oder dreiwertiger Rest eines geradkettigen oder verzweigten, unsubstituierten oder substituierten (C₁-C₁₈)Alkans oder Cycloalkans, eines ein- oder mehrkernigen (C₆-C₁₄)Aromaten oder eines (C₄-C₁₂)Heteroaromaten mit Sauerstoff, Schwefel oder Stickstoff als Heteroatom,
ist und
n eine ganze Zahl von 1 bis 3 und
m 2 oder 3 bedeutet.

2. Strahlungsempfindliches Gemisch gemäß Anspruch 1, dadurch gekennzeichnet, daß R¹ ein Phenyl-, Naphthyl-, Thiophen-2- oder -3-yl, Benzo[b]thiophen-2-oder 3-yl oder ein Phenylrest ist, an den ein fünf- oder sechsgliedriger Ring mit 1 bis 3 Heteroatomen, wie Sauerstoff, Stickstoff oder Schwefel ankondensiert ist, vorzugsweise ein Chromanyl-, Chromenyl-, Thiochromanyl-, Isochromanyl- oder Isothiochromanyl, Indolinyl-, Benzothiazolyl-, Chinolinyl-, 1,2,3,4-Tetrahydrochinolinyl- oder Isochinolinylrest.

3. Strahlungsempfindliches Gemisch gemäß Anspruch 1 oder 2, dadurch gekennzeichnet, daß der Rest R¹ mit ein bis drei gleichartigen oder verschiedenen Substituenten, wie Fluor-, Chlor- oder Bromatomen, (C₁-C₆)Alkyl-, (C₂-C₆)Alkenyl-, (C₂-C₄)Alkinyl-, (C₁-C₆)Alkoxy-, (C₆-C₁₀)Aryl-, (C₆-C₁₀)Aryloxy-, (C₇-C₁₄)Aralkyl-, (C₇-C₁₄)Aryloxyalkyl-, (C₇-C₁₄)-Aryloxyalkoxy-, (C₁-C₆)Alkylthio-, (C₆-C₁₀)Arylthio-, (C₁-C₄)-Dialkylamino-, (C₁-C₆)-Carboxyalkyl-, Cyano-, Nitro-, (C₁-C₁₀)Alkansulfonyl-, (C₆-C₁₄)-Arylsulfonylgruppen oder den Gruppen R³-SO₂-O-, R³-SO₂-NH-, HO-C(CF₃)-R²- oder R³-SO₂-O-C(CF₃)R²- substituiert ist, wobei R² und R³ die im Anspruch 1 angegebene Bedeutung haben.

4. Strahlungsempfindliches Gemisch gemäß Anspruch 1, dadurch gekennzeichnet, daß der Rest R² ein Trifluormethylrest ist.

5. Strahlungsempfindliches Gemisch gemäß Anspruch 1, dadurch gekennzeichnet, daß der Rest R³ in den Verbindungen der Formel I mit n = 1 sowie in den Verbindungen der Formel II ein (C₁-C₆)Alkylrest, wie Methyl, Ethyl, Propyl, Isopropyl, oder Butyl, ein Cycloalkylrest, wie Cyclohexyl, ein per- oder hochfluorierten (C₁-C₆)Alkylrest, wie Trifluormethyl, ein (C₆-C₁₀)Arylrest, wie Phenyl, Tolyl, Naphthyl, Dihydro- oder Tetrahydronaphthyl, (C₇-C₁₄)Arylalkylrest, wie Benzyl, oder ein (C₄-C₉)Heteroarylrest, wie Thiophen-2- oder -3-yl, und in den Verbindungen der Formel II mit n = 2 ein (C₁-C₁₀)Alkylen-, insbesondere Methylen, Ethan-1,2-diyl oder Propan-1,3-diyl, oder ein (C₆-C₁₀)Arylen, wie ortho-, meta- oder para-Phenylen oder Naphthalindiyl ist.

6. Strahlungsempfindliches Gemisch gemäß Anspruch 5, dadurch gekennzeichnet, daß der Rest R³ nicht mehr als 15 Kohlensstoffatome aufweist.

7. Strahlungsempfindliches Gemisch gemäß einem oder mehreren der Ansprüche 1 bis 6, dadurch gekennzeichnet, daß der Anteil der Verbindung der allgemeinen Formel I und/oder II 0,5 bis 25 Gew.-%, bevorzugt bei 1 bis 10 Gew.-%, jeweils bezogen auf das Gesamtgewicht der Feststoffe, beträgt.

8. Strahlungsempfindliches Gemisch gemäß einem oder mehreren der Ansprüche 1 bis 7, dadurch gekennzeichnet, daß der Gehalt an Bindemittel 1 bis 95 Gew.-%, vorzugsweise 5 bis 90 Gew.-%,, besonders bevorzugt 30 bis 85 Gew.-%, jeweils bezogen auf das Gesamtgewicht der Feststoffe, beträgt.

9. Strahlungsempfindliche Gemische gemäß Ansprüchen 1 bis 4, dadurch gekennzeichnet, daß der Anteil an säurespaltbaren Verbindungen in dem positiv arbeitenden Gemisch 1 bis 50 Gew.-%, bevorzugt 5 bis 40 Gew.-%, jeweils bezogen auf das Gesamtgewicht der Feststoffe, beträgt.

10. Strahlungsempfindliches Gemisch gemäß einem oder mehreren der Ansprüche 1 bis 8, dadurch gekennzeichnet, daß der Gehalt an säurevernetzbaren Verbindungen in dem negativ arbeitenden Gemisch 1 bis 50 Gew.-%, vorzugsweise bei 5 bis 40 Gew.-%, jeweils bezogen auf das Gesamtgewicht der Feststoffe, beträgt.

11. Positiv oder negativ arbeitendes strahlungsempfindliches Aufzeichnungsmaterial mit einem Träger und einer strahlungsempfindlichen Schicht, dadurch gekennzeichnet, daß die Schicht aus dem strahlungsempfindlichen Gemisch gemäß einem oder mehreren der Ansprüche 1 bis 10 besteht.

12. Verwendung von Sulfonsäureestern der Formel
[R¹-CR²(CF₃)-O-SO₂-]ₙR³ (I)
oder
R¹[-CR²(CF₃)-O-SO₂-R³]ₘ (II),
als Photosäuregeneratoren in einem strahlungsempfindlichen Gemisch, worin R¹, R², R³, n und m die im Anspruch 1 angegebene Bedeutung haben.

## Claims

1. A radiation-sensitive, positive-working or negative-working mixture comprising
a) a compound forming a strong acid on irradiation,
b) either, for a positive-working mixture, a compound having at least one acid-cleavable C-O-C or C-O-Si bond or, for a negative-working mixture, a compound having at least two acid-crosslinkable groups and
c) a binder which is insoluble in water but soluble or at least swellable in aqueous alkaline solutions,
wherein the compound a) is a sulfonic acid ester of the formula
[R¹-CR²(CF₃)-O-SO₂-]ₙR³ (I)
or
R¹[-CR²(CF₃)-O-SO₂-R³]ₘ (II)
in which
R¹, in the compounds of the formula I, is a mononuclear or polynuclear unfused or fused (C₆-C₁₄)aryl or (C₄-C₁₁)heteroaryl radical containing oxygen, sulfur or nitrogen as a heteroatom and, in the compounds of the formula II, is a divalent or trivalent radical of a mononuclear or polynuclear unfused or fused (C₆-C₁₄)aromatic or (C₄-C₁₂)heteroaromatic containing oxygen, sulfur or nitrogen as a heteroatom or a divalent radical of the formula -C₆H₄-X-C₆H₄-, in which X is an oxygen atom, a carbonyl or sulfonyl group or a group CR⁷R⁸, in which R⁷ and R⁸ are identical or different and are a methyl or trifluoromethyl radical,
R² is a hydrogen atom or a methyl, trifluoromethyl or cyano radical,
R³, in the compounds of the formula I where n = 1 and in the compounds of the formula II, is a straight-chain or branched, unsubstituted or substituted (C₁-C₁₈)alkyl radical, a (C₄-C₁₀)-cycloalkyl radical, a (C₂-C₆)alkenyl radical, an unsubstituted or substituted (C₆-C₁₄)aryl or (C₄-C₁₂)heteroaryl radical containing oxygen, sulfur or nitrogen as a heteroatom or a (C₇-C₁₈)aralkyl radical and,
in the compounds of the formula I where n = 2 or 3, is a divalent or trivalent radical of a straight-chain or branched, unsubstituted or substituted (C₁-C₁₈)alkane or cycloalkane, of a mononuclear or polynuclear (C₆-C₁₄)aromatic or of a (C₄-C₁₂)heteroaromatic containing oxygen, sulfur or nitrogen as a heteroatom,
and
n is an integer from 1 to 3 and
m is 2 or 3.

2. The radiation-sensitive mixture as claimed in claim 1, wherein R¹ is a phenyl, naphthyl, thiophen-2-yl, thiophen-3-yl, benzo[b]thiophen-2-yl or benzo[b]thiophen-3-yl or a phenyl radical to which a five-membered or six-membered ring having 1 to 3 heteroatoms, such as oxygen, nitrogen or sulfur, is fused, preferably a chromanyl, chromenyl, thiochromanyl, isochromanyl or isothiochromanyl, indolinyl, benzothiazolyl, quinolyl, 1,2,3,4-tetrahydroquinolyl or isoquinolyl radical.

3. The radiation-sensitive mixture as claimed in claim 1 or 2, wherein the radical R¹ is substituted by one to three identical or different substituents, such as fluorine, chlorine or bromine atoms, (C₁-C₆)alkyl, (C₂-C₆)alkenyl, (C₂-C₄)alkynyl, (C₁-C₆)alkoxy, (C₆-C₁₀)aryl, (C₆-C₁₀)aryloxy, (C₇-C₁₄)aralkyl, (C₇-C₁₄)aryloxyalkyl, (C₇-C₁₄)aryloxyalkoxy, (C₁-C₆)alkylthio, (C₆-C₁₀)arylthio, (C₁-C₄)dialkylamino, (C₁-C₆)carboxyalkyl, cyano, nitro, (C₁-C₁₀)alkanesulfonyl or (C₆-C₁₄)arylsulfonyl groups or the groups R³-SO₂-O-, R³-SO₂-NH-, HO-C(CF₃)-R²- or R³-SO₂-O-C(CF₃)R²-, in which R² and R³ have the meaning stated in claim 1.

4. The radiation-sensitive mixture as claimed in claim 1, wherein the radical R² is a trifluoromethyl radical.

5. The radiation-sensitive mixture as claimed in claim 1, wherein the radical R³, in the compounds of the formula I where n = 1 and in the compounds of the formula II, is a (C₁-C₆)alkyl radical, such as methyl, ethyl, propyl, isopropyl or butyl, a cycloalkyl radical, such as cyclohexyl, a perfluorinated or highly fluorinated (C₁-C₆)alkyl radical, such as trifluoromethyl, a (C₆-C₁₀)aryl radical, such as phenyl, tolyl, naphthyl or dihydronaphthyl or tetrahydronaphthyl, a (C₇-C₁₄)arylalkyl radical, such as benzyl, or a (C₄-C₉)heteroaryl radical, such as thiophen-2-yl or thiophen-3-yl, and, in the compounds of the formula II where n = 2, is a (C₁-C₁₀)alkylene, in particular methylene, ethane-1,2-diyl or propane-1,3-diyl, or a (C₆-C₁₀)arylene, such as ortho-, meta- or paraphenylene or naphthalenediyl.

6. The radiation-sensitive mixture as claimed in claim 5, wherein the radical R³ has not more than 15 carbon atoms.

7. The radiation-sensitive mixture as claimed in one or more of claims 1 to 6, wherein the amount of the compound of the formula I and/or II is 0.5 to 25% by weight, preferably 1 to 10% by weight, based in each case on the total weight of the solids.

8. The radiation-sensitive mixture as claimed in one or more of claims 1 to 7, wherein the content of binder is 1 to 95% by weight, preferably 5 to 90% by weight, particularly preferably 30 to 85 % by weight, based in each case on the total weight of the solids.

9. The radiation-sensitive mixture as claimed in any of claims 1 to 4, wherein the amount of acid-cleavable compounds in the positive-working mixture is 1 to 50% by weight, preferably 5 to 40% by weight, based in each case on the total weight of the solids.

10. The radiation-sensitive mixture as claimed in one or more of claims 1 to 8, wherein the content of acid-crosslinkable compounds in the negative-working mixture is 1 to 50% by weight, preferably 5 to 40% by weight, based in each case on the total weight of the solids.

11. A positive-working or negative-working radiation-sensitive recording material having a substrate and a radiation-sensitive layer, wherein the layer comprises the radiation-sensitive mixture as claimed in one or more of claims 1 to 10.

12. The use of sulfonic acid esters of the formula
[R¹-CR²(CF₃)-O-SO₂-]ₙR³ (I)
or
R¹[-CR²(CF₃)-O-SO₂-R³]ₘ (II)
as photosensitive acid generators in a radiation-sensitive mixture, where R¹, R², R³, n and m have the meanings stated in claim 1.

## Revendications

1. Mélange sensible au rayonnement positif ou négatif
a) avec un composé formant par irradiation un acide fort,
b) soit, pour un mélange positif, avec un composé comportant au moins une liaison C-O-C- ou C-O-Si-dissociable par un acide, soit, pour un mélange négatif, un composé avec au moins deux groupes réticulables par un acide, et
c) un liant insoluble dans l'eau, en revanche soluble ou au moins gonflable en solutions aqueuses alcalines,
caractérisé en ce que le composé a) est un ester d'acide sulfonique de formules
[R¹-CR²(CF₃)-O-SO₂-]ₙR³ (I)
ou
R¹-[CR²(CF₃)-O-SO₂-R³]ₘ (II)
dans laquelle
R¹ dans les composés de formule I représente un reste aryle en C₆-C₁₄ ou hétéroaryle en C₄-C₁₁, comportant des atomes d'oxygène, de soufre ou d'azote en tant qu'hétéroatomes, mono- ou polycyclique, non condensé ou condensé, et dans les composés de formule II un reste bi- ou trifonctionnel d'un hétéroaromatique en C₄-C₁₂, comportant des atomes d'oxygène, de soufre ou d'azote en tant qu'hétéroatomes, ou d'un aromatique en C₆-C₁₄ mono- ou polycyclique, non condensé ou condensé, ou un reste bifonctionnel de formule -C₆H₄-X-C₆H₄-, dans laquelle X représente un atome d'oxygène, un groupe carbonyle ou sulfonyle, ou un groupe CR⁷R⁸ dans lequel R⁷ et R⁸ sont identiques ou différents et représentent un reste méthyl- ou trifluorométhyle,
R² représente un atome d'hydrogène, un reste méthyle, trifluorométhyle ou cyano,
R³ dans les composés de formule I avec n = 1 ainsi que dans les composés de formule II, représente un reste cycloalkyle en C₄-C₁₀, un reste alcényle en C₂-C₆, un reste alkyle en C₁-C₁₈ linéaire ou ramifié, non substitué ou substitué, un reste aryle en C₆-C₁₄ ou un reste hétéroaryle en C₄-C₁₂, comportant des atomes d'oxygène, de soufre ou d'azote en tant qu'hétéroatomes, non substitué ou substitué, ou un reste aralkyle en C₇-C₁₈, dans les composés de formule I avec n = 2 ou 3, représente un reste bi- ou trifonctionnel d'un cycloalcane ou d'un alcane en C₁-C₁₈ linéaire ou ramifié, non substitué ou substitué, d'un aromatique en C₆-C₁₄ ou d'un hétéroaromatique en C₄-C₁₂ mono- ou polycyclique comportant des atomes d'oxygène, de soufre ou d'azote en tant qu'hétéroatomes,
et
n est un nombre entier de 1 à 3, et
m vaut 2 ou 3.

2. Mélange sensible au rayonnement selon la revendication 1, caractérisé en ce que R¹ est un reste phényle, naphtyle, thiophène-2- ou -3-yle, benzo[b]thiophène-2- ou -3-yle, ou un phényle, sur lequel est condensé un cycle à 5 ou 6 chaînons avec 1 à 3 hétéroatomes tels que des atomes d'oxygène, d'azote, ou de soufre, de préférence un reste chromanyle, chroményle, thiochromanyle, isochromanyle, ou isothiochromanyle, indolyle, benzothiazolyle, quinolinyle, 1,2,3,4-tétrahydroquinolinyle ou isoquinolinyle.

3. Mélange sensible au rayonnement selon la revendication 1 ou 2, caractérisé en ce que le reste R¹ est substitué par 1 à 3 substituants identiques ou différents, tels que des atomes de fluor, de chlore ou de brome, des groupes alkyle en C₁-C₆, alcényle en C₂-C₆, alcynyle en C₂-C₄, alkoxy en C₁-C₆, aryle en C₆-C₁₀, aryloxy en C₆-C₁₀, aralkyle en C₇-C₁₄, aryloxyalkyle en C₇-C₁₄, aryloxyalkoxy en C₇-C₁₄, alkylthio en C₁-C₆, arylthio en C₆-C₁₀, di(alkyle en C₁-C₄)amino, carboxyalkyle en C₁-C₆, cyano, nitro, (alcane en C₁-C₁₀)-sulfonyle, (aryle en C₆-C₁₄)-sulfonyle, ou est substitué par des groupes R³-SO₂-O-, R³-SO₂-NH-, HO-C(CF₃)-R²- ou R³-SO₂-O-C(CF₃)R²-, R² et R³ ayant la signification donnée dans la revendication 1.

4. Mélange sensible au rayonnement selon la revendication 1, caractérisé en ce que le reste R² est un reste trifluorométhyle.

5. Mélange sensible au rayonnement selon la revendication 1, caractérisé en ce que le reste R³ dans les composés de formule I avec n = 1, ainsi que dans les composés de formule II, représente de préférence un reste alkyle en C₁-C₆ tel que méthyle, éthyle, propyle, isopropyle ou butyle, un reste cycloalkyle tel que cyclohexyle, un reste alkyle en C₁-C₆ perfluoré ou hautement fluoré, tel que trifluorométhyle, un reste aryle en C₆-C₁₀, tel que phényle, tolyle, naphtyle, dihydro- ou tétrahydronaphtyle, un reste aralkyle en C₇-C₁₄, tel que benzyle, ou un reste hétéroaryle en C₄-C₉, tel que thiophène-2- ou -3-yle, et dans les composés de formule II avec n = 2, un reste alkylène en C₁-C₁₀, plus particulièrement méthylène, éthane-1,2-diyle ou propane-1,3-diyle, ou un reste arylène en C₆-C₁₀, tel que ortho-, méta- ou paraphénylène ou naphtalènediyle.

6. Mélange sensible au rayonnement selon la revendication 5, caractérisé en ce que le reste R³ ne présente pas plus de 15 atomes de carbone.

7. Mélange sensible au rayonnement selon une ou plusieurs des revendications 1 à 6, caractérisé en ce que le taux en composé de formule générale I et/ou II est de 0,5 à 25 % en poids, de préférence de 1 à 10 % en poids, à chaque fois par rapport à la totalité du poids de l'extrait sec.

8. Mélange sensible au rayonnement selon une ou plusieurs des revendications 1 à 7, caractérisé en ce que la teneur en liant est de 1 à 95 % en poids, de préférence de 5 à 90 % en poids, de manière particulièrement préférée de 30 à 85 % en poids, à chaque fois par rapport au poids total de l'extrait sec.

9. Mélange sensible au rayonnement selon les revendications 1 à 4, caractérisé en ce que le taux en composés dissociables avec un acide dans le mélange positif est de 1 à 50 % en poids, de préférence de 5 à 40 % en poids, à chaque fois par rapport au poids total de l'extrait sec.

10. Mélange sensible au rayonnement selon une ou plusieurs des revendications 1 à 8, caractérisé en ce que la teneur en composés réticulables par un acide dans le mélange négatif est de 1 à 50 % en poids, de préférence de 5 à 40 % en poids, à chaque fois par rapport au poids total de l'extrait sec.

11. Matière d'enregistrement sensible au rayonnement positive ou négative avec un support et une couche sensible au rayonnement, caractérisée en ce que la couche en mélange sensible au rayonnement présente une composition selon une ou plusieurs des revendications 1 à 10.

12. Utilisation des esters d'acide sulfonique de formules
[R¹-CR²(CF₃)-O-SO₂-]ₙR³ (I)
ou
R¹-[CR²(CF₃)-O-SO₂-R³]ₘ (II)
en tant que photorégénérateurs d'acide dans un mélange sensible au rayonnement, où R¹, R², R³, n et m ont les significations données dans la revendication 1.
